(19) Europäisches Patentamt
European Patent Office
Office européen des brevets

(11) Veröffentlichungsnummer: **0 049 855**
**B1**

(12) # EUROPÄISCHE PATENTSCHRIFT

(45) Veröffentlichungstag der Patentschrift: **29.01.86**

(51) Int. Cl.⁴: **C 07 D 501/20,** C 07 D 498/04, A 61 K 31/545 // C07D293/06

(21) Anmeldenummer: **81107991.2**

(22) Anmeldetag: **06.10.81**

(54) **Neue Cephalosporinderivate, Verfahren zu ihrer Herstellung und Zwischenprodukte dafür sowie entsprechende pharmazeutische Präparate.**

(30) Priorität: **10.10.80 CH 7583/80**
**18.08.81 CH 5335/81**

(43) Veröffentlichungstag der Anmeldung: **21.04.82 Patentblatt 82/16**

(45) Bekanntmachung des Hinweises auf die Patenterteilung: **29.01.86 Patentblatt 86/5**

(84) Benannte Vertragsstaaten: **CH DE FR GB IT LI**

(56) Entgegenhaltungen:
**FR - A - 2 137 899**
**FR - A - 2 346 014**

**Die Akte enthält technische Angaben, die nach dem Eingang der Anmeldung eingereicht wurden und die nicht in dieser Patentschrift enthalten sind.**

(73) Patentinhaber: **F. HOFFMANN-LA ROCHE & CO. Aktiengesellschaft, CH-4002 Basel (CH)**

(72) Erfinder: **Reiner, Roland, Dr., Rheinfelderstrasse 8, CH-4058 Basel (CH)**
Erfinder: **Weiss, Urs, Wyhlenstrasse 20A, CH-4133 Pratteln (CH)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

**EP 0 049 855 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Acylderivate, und zwar Cephalosporinderivate der allgemeinen Formel

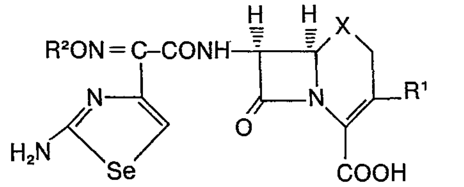

I

in der $R^1$ Wasserstoff, Methyl, Methoxy, Chlor oder eine Gruppe $-CH_2Y$ darstellt, worin Y einen in der Cephalosporin-Chemie üblichen Rest einer nucleophilen Verbindung bedeutet, worin ferner $R^2$ Wasserstoff oder eine $C_{1-4}$-Alkylgruppe darstellt, die durch die Gruppe $COOR^3$ substituiert sein kann, worin $R^3$ Wasserstoff, ein Kation einer Base oder eine leicht hydrolysierbare Estergruppe bedeutet, und worin X Schwefel, Sauerstoff oder eine der Gruppen $-SO-$ und $-SO_2-$ darstellt, sowie leicht hydrolysierbare Ester, leicht hydrolysierbare Äther und Salze dieser Verbindungen und Hydrate der Verbindungen der Formel I bzw. von deren Estern, Äthern und Salzen.

Als Reste einer nucleophilen Verbindung (Y-Reste) kommen beispielsweise in Betracht: Acetoxy, Carbamoyloxy oder Reste der allgemeinen Formel

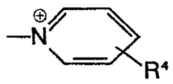

worin $R^4$ Wasserstoff oder Carbamoyl darstellen; oder eine Gruppe $-SR^5$ worin $R^5$ einen ggf. substituierten 5- oder 6-gliedrigen Heterocyclus mit 1–4 Heteroatomen, z.B. Sauerstoff, Schwefel, Selen und/oder Stickstoff. Beispiele für $R^5$ sind Tetrazolyl, Triazolyl, Thiadiazolyl und Triazinyl. Diese Reste können auch substituiert sein, z.B. durch $C_{1-4}$-Alkyl, wie Methyl oder Äthyl, durch Halogen, wie Chor oder Brom, durch Hydroxy oder Oxo-gruppen. Beispiele solcher substituierten Reste sind die

1-Methyl-tetrazol-5-yl-,
2-Methyl-1,3,4-thiadiazol-5-yl,
1,4,5,6-Tetrahydro-4-methyl-5,6-dioxo-as-triazin-3-yl-,
2,5-Dihydro-6-hydroxy-2-methyl-5-oxo-as-triazin-3-yl
und die
1,2,5,6-Tetrahydro-2-methyl-5,6-dioxo-as-triazin-2-ylgruppe.

$R^2$ kann ausser Wasserstoff $C_{1-4}$ Alkyl bedeuten, z.B. Methyl, Äthyl, n-Propyl, Isopropyl, n-Butyl, oder auch $COOR^3-C_{1-4}$-Alkyl, worin $R^3$ Wasserstoff, ein Kation einer Base oder eine leicht hydrolysierbare Estergruppe darstellt. Diese Bedeutungen werden nachstehend näher erläutert.

Im Rest «$COOR^3-C_{1-4}$-Alkyl» kann sich die Carboxygruppe an beliebiger Stelle des Alkylrestes befinden, wie z.B. in 1-Carboxyäthyl, 2-Carboxyäthyl, 1-Carboxy-1-methoxy-äthyl, 2-Carboxy-1-methyl-äthyl, 1-Carboxy-1-methyl-n-propyl, Carboxymethyl. Bevorzugt ist 1-Carboxy-methyl-äthyl.

Als leicht hydrolysierbare Ester der Verbindungen der Formel I sind Verbindungen der Formel I zu verstehen, deren Carboxygruppe(n) (d.h. die 3-Carboxygruppe und/oder die Carboxygruppe einer Carboxy-$C_{1-4}$-Alkylgruppe $R^2$) in Form von leicht hydrolysierbaren Estergruppen vorliegen. Beispiele solcher Ester, die herkömmlicher Art sein können, sind die niederen Alkanoyloxyalkylester, z.B. der Acetoxymethyl-, Pivaloyloxymethyl-, 1-Acetoxyäthyl- und 1-Pivaloyloxyäthylester; die niederen Alkoxycarbonyloxyalkylester, z.B. der Methoxycarbonyloxymethyl-, 1-Äthoxycarbonyloxyäthyl- und 1-Isopropoxycarbonyloxyäthylester; die Lactonylester, z.B. der Phthalidyl- und Thiophthalidylester; die niederen Alkoxymethylester, z.B. der Methoxymethylester; und die niederen Alkanoylaminomethylester, z.B. der Acetamidomethylester. Auch andere Ester, z.B. die Benzyl- und Cyanmethylester, können brauchbar sein.

Als leicht hydrolysierbare Äther der Verbindungen der Formel I sind Verbindungen der Formel I zu verstehen, worin $R^1$ einen Rest $-CH_2SR^5$ mit einem durch eine Hydroxygruppe substituierten Heterocyclus $R^5$ darstellt, dessen Hydroxygruppe in Form einer leicht hydrolysierbaren Äthergruppe vorliegt. Ein Beispiel eines solchen hydroxysubstituierten Heterocyclus $R^5$ ist die 2,5-Dihydro-6-hydroxy-2-methyl-5-oxo-as-triazin-3-yl-Gruppe.

Als Äthergruppen kommen die gleichen Gruppen in Betracht, wie sie oben bereits für die leicht hydrolysierbaren Estergruppen erwähnt wurden. Vertreter solcher Äther sind also beispielsweise die niederen Alkanoyloxyalkyläther, z.B.
der Acetoxymethyl-, Pivaloyloxymethyl-,
1-Acetoxyäthyl- und 1-Pivaloyloxyäthyläther;
die niederen Alkoxycarbonyloxyalkyläther, z.B.
der Methoxycarbonyloxymethyl-,
1-Äthoxycarbonyloxyäthyl- und
I-Isopropoxycarbonyloxyäthyläther;
die Lactonyläther, z.B.
der Phthalidyl- und Thiophthalidyläther;
die niederen Alkoxymethyläther, z.B.
der Methoxymethyläther;
und die niederen Alkanoylaminomethyläther, z.B.
der Acetamidomethyläther.

Beispiele von Salzen der Verbindungen der Formel I sind Alkalimetallsalze, wie das Natrium- und Kaliumsalz; das Ammoniumsalz; Erdalkalimetallsalze, wie das Calciumsalz; Salze mit organischen Basen, wie Salze mit Aminen, z.B. Salze mit N-Äthyl-piperidin, Procain, Dibenzylamin, N,N'-Dibenzyläthylendiamin, Alkylaminen oder Dialkylaminen, sowie Salze mit Aminosäuren, wie z.B. Salze mit Arginin oder Lysin. Die Salze können Monosalze oder auch Di- oder Trisalze sein. Die weiteren Salzbildungen können in Verbindungen mit dem Hydroxyrest eines hydroxysubstituierten Heterocyclus $R^5$ bzw. mit der Carboxygruppe einer Carboxy-$C_{1-4}$-Alkylgruppe $R^2$ auftreten.

Die Verbindungen der Formel I bilden ebenfalls Additionssalze mit organischen oder anorganischen Säuren. Beispiele solcher Salze sind Hydrohalogenide, beispielsweise Hydrochloride, Hydrobromide, Hydrojodide, sowie andere Mineralsäuresalze, wie Sulfate, Nitrate, Phosphate u. dgl., Alkyl- und Mono-arylsulfonate, wie Äthansulfonate, Toluolsulfonate, Benzolsulfonate u. dgl. und auch andere organische Säuresalze, wie Acetate, Tartrate, Maleate, Citrate, Benzoate, Salicylate, Ascorbate u. dgl.

Die Verbindungen der Formel I (einschliesslich deren Salze, leicht hydrolysierbare Ester und Äther) können hydratisiert sein. Die Hydratisierung kann im Zuge des Herstellungsverfahrens erfolgen oder allmählich als Folge hygroskopischer Eigenschaften eines zunächst wasserfreien Produktes auftreten.

Die erfindungsgemässen Produkte können in der syn-isomeren Form

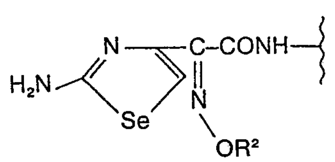

oder in der anti-isomeren Form

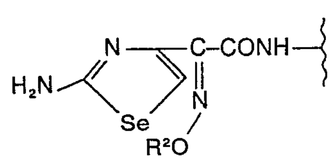

bzw. als Gemische dieser beiden Formen vorliegen. Bevorzugt ist die syn-isomere Form bzw. Gemische, in denen die syn-isomere Form überwiegt.

Bevorzugte Produkte sind:

(6R,7R)-7-[2-(2-Amino-2-selenazolyl)-2-[(Z)-methoxyimino]-acetamido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure

(6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure-5-oxid

(6R,7R)-7-[2-(2-Amino-4-selenazolyl)-2-[(Z)-methoxyimino]-acetamido]-3-methyl-8-oxo-5-thia-1-azabicyclo[4.2.0]oct-2-en-2-carbonsäure

Methylen (6R,7R)-7-[2-(2-Amino-4-selenazolyl)-2-[(Z)-methoxyimino]acetamido]-3-methyl-8-oxo-5-thia-1-azabicyclo[4.2.0]oct-2-en-2-carboxylat-pivalat

(6R,7R)-7-[2-(2-Amino-4-selenazolyl-2-[(Z)-methoxyimino]-acetamido]-3-[[(5-methyl-1,3,4-thiadiazol-2-yl)thio]methyl]-8-oxo-5-thia-1-azabicyclo[4.2.0]oct-2-en-2-carbonsäure

(6R,7R)-7-[2-(2-Amino-4-selenazolyl-2-[(Z)-methoxyimino]-acetamido]-3-[[(1-methyl-1H-tetrazol-5-yl)-thio]-methyl]-8-oxo-5-thia-1-azabicyclo[4.2.0]oct-2-en-2-carbonsäure

(6R,7R)-7-[2-(2-Amino-4-selenazolyl)-2-[(Z)-methoxyimino]-acetamido]-8-oxo-3-[[(1,4,5,6-tetrahydro-4-methyl-5,6-dioxo-as-triazin-3-yl)thio]methyl]-5-thia-1-azabicyclo[4.2.0]-oct-2-en-2-carbonsäure

(6R,7R)-3-(Acetoxymethyl)-7-[2-(2-amino-4-selenazolyl)-2-[(Z)-methoxyimino]-acetamido]-8-oxo-5-thia-1-azabicyclo[4.2.0]oct-2-en-2-carbonsäure

(6R,7R)-3-(Acetoxymethyl)-7-[(Z)-2-(2-amino-4-selenazolyl)-2-[(1-carboxy-1-methyläthoxy)imino]acetamido]-8-oxo-5-thia-1-azabicyclo-[4.2.0]oct-2-en-carbonsäure

1-[[(6R,7R)-7-[(Z)-2-(2-Amino-4-selenazolyl)-2-[(1-carboxy-1-methyläthoxy)imino]acetamido]-2-carboxy-8-oxo-5-thia-1-azabicyclo-[4.2.0]oct-2-en-3-yl]methyl]pyridinium-hydroxid (inneres Salz)

und deren Salze sowie entsprechende Hydrate.

Die obigen Acylderivate werden erfindungsgemäss dadurch hergestellt, dass man

a) eine Verbindung der allgemeinen Formel

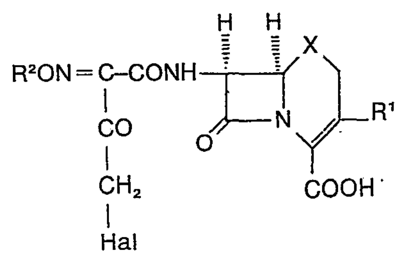

II

in der $R^1$, $R^2$ und X die oben gegebene Bedeutung haben,

Hal ein Halogenatom darstellt und die Carboxygruppe in geschützter Form vorliegen kann, oder ein Salz dieser Verbindung mit Selenharnstoff umsetzt und gegebenenfalls eine allenfalls vorhandene Carboxyschutzgruppe abspaltet, oder dass man

b) aus einer Verbindung der allgemeinen Formel

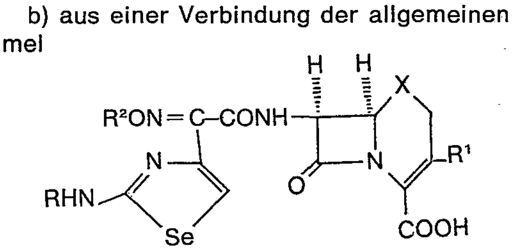

III

in der $R^1$, $R^2$ und X die oben gegebene Bedeutung haben, R eine abspaltbare Schutzgruppe darstellt und die Carboxygruppe in geschützter Form vorliegen kann,

oder aus einem Salz dieser Verbindung die Schutzgruppe R und gegebenenfalls eine allenfalls vorhandene Carboxyschutzgruppe abspaltet, oder dass man

c) zur Herstellung von Verbindungen der Formel I, worin $R^1$ die Gruppe $-CH_2SR^5$ darstellt, worin $R^5$ die oben gegebene Bedeutung hat, eine Verbindung der allgemeinen Formel

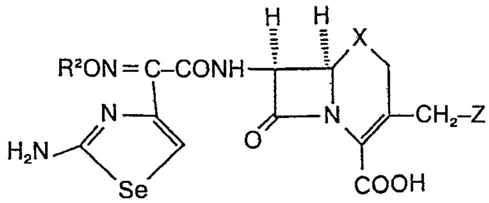

IV

in der $R^2$ und X die oben gegebene Bedeutung haben, Z eine austretende Gruppe darstellt und die Carboxygruppe durch Salzbildung mit einer anorganischen oder tertiären organischen Base geschützt sein kann,
oder ein Salz dieser Verbindung in Gegenwart von Wasser mit einem Thiol der allgemeinen Formel

$$HS–R^5 \qquad V$$

in der $R^5$ die oben gegebene Bedeutung hat, umsetzt, und gegebenenfalls eine allenfalls vorhandene Carboxyschutzgruppe abspaltet, oder dass man

d) zur Herstellung von Verbindungen der Formel I, worin X eine der Gruppen $-SO-$ und $-SO_2-$ darstellt, eine Verbindung der Formel I, worin X Schwefel oder die Gruppe $-SO-$ darstellt, oder ein Salz dieser Verbindung oxidiert, oder dass man,

e) zur Herstellung eines leicht hydrolysierbaren Esters bzw. Äthers einer Verbindung der Formel I eine Carbonsäure bzw. ein Enol der Formel I einer entsprechenden Veresterung bzw. Verätherung unterwirft, oder dass man

f) zur Herstellung von Salzen oder Hydraten einer Verbindung der Formel I bzw. Hydraten dieser Salze eine Verbindung der Formel I in ein Salz oder Hydrat bzw. ein Hydrat dieses Salzes überführt.

Die in den Ausgangsverbindungen der Formeln II und III vorhandene Carboxygruppe in 3-Stellung kann wahlweise geschützt sein, z.B. durch Veresterung zu einem leicht spaltbaren Ester, wie dem Silylester, z.B. dem Trimethylsilylester. Es kommen auch die oben erläuterten, leicht hydrolysierbaren Ester in Betracht. Die Carboxygruppe kann auch durch Salzbildung mit einer anorganischen oder tertiären organischen Base, wie Triäthylamin, geschützt werden.

Die erfindungsgemässe Umsetzung des Halogenids der Formel II bzw. eines Salzes davon mit Selenharnstoff gemäss Variante a) des erfindungsgemässen Verfahrens erfolgt vorzugsweise in einem inerten Lösungsmittel, wie z.B. in einem niederen Alkanol, z.B. Äthanol, in einem niederen Keton, wie Aceton, in einem Äther, wie Tetrahydrofuran oder Dioxan, in Dimethylformamid, Dimethylacetamid, in Wasser oder in Mischungen davon. Die Reaktionstemperatur liegt im allgemeinen im Bereich von etwa 0°C bis 60°C, vorzugsweise wird bei Zimmertemperatur gearbeitet. Als Halogenid der Formel II kann das Chlorid, Bromid, Fluorid oder Jodid eingesetzt werden, bevorzugt verwendet man das Chlorid oder das Bromid. Es kann die freie Säure der Formel III eingesetzt werden, wahlweise aber auch ein Salz davon, wobei die gleichen Salze wie die oben erläuterten Salze der Verbindungen der Formel I in Betracht kommen.

Nach Durchführung der Verfahrensvariante a) kann, falls erwünscht, eine allenfalls im Reaktionsprodukt vorhandene Carboxyschutzgruppe abgespalten werden. Wenn die Schutzgruppe eine Silylgruppe darstellt (Silylester), kann diese Gruppe besonders leicht durch Behandeln des Umsetzungsproduktes mit Wasser abgespalten werden. Niedere Alkanoyloxyalkyl-, Alkoxycarbonyloxyalkyl-, Lactonyl-, Alkoxymethyl- und Alkanoylaminomethylester werden vorzugsweise enzymatisch mit Hilfe einer geeigneten Esterase (bei etwa 20–40°C) abgespalten. Wenn die Carboxylgruppe durch Salzbildung (z.B. mit Triäthylamin) geschützt ist, so kann die Abspaltung dieser salzbildenden Schutzgruppe durch Behandlung mit Säure erfolgen. Als Säure kann hierbei z.B. Salzsäure, Schwefelsäure, Phosphorsäure oder Citronensäure verwendet werden.

Die Schutzgruppen R in den für die Variante b) des erfindungsgemässen Verfahrens einsetzbaren Ausgangsverbindungen der Formel III sind beispielsweise sauer-hydrolytisch abspaltbare Schutzgruppen, wie z.B. t-Butoxycarbonyl oder Trityl, oder auch basisch-hydrolytisch abspaltbare Schutzgruppen, wie z.B. Trifluoracetyl. Bevorzugte R-Schutzgruppen sind Chlor-, Brom- und Jodacetyl, insbesondere Chloracetyl. Letztere Schutzgruppen können durch Behandeln mit Thioharnstoff abgespalten werden.

Die Ausgangsverbindungen der Formel III können z.B. durch N-Acylierung der entsprechenden 7-Aminoverbindung hergestellt werden, und zwar indem man eine Verbindung der allgemeinen Formel

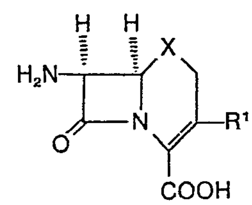

VI

in der X und $R^1$ die oben gegebene Bedeutung haben und die Carboxygruppe und/oder die Aminogruppe in geschützter Form vorliegen kann, mit einer Säure der allgemeinen Formel

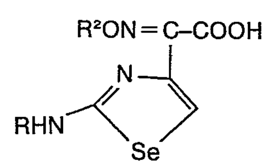

VII

in der R und $R^2$ die oben gegebene Bedeutung haben,
oder mit einem reaktionsfähigen funktionellen Derivat dieser Säure umsetzt und eine allenfalls vorhandene Carboxyschutzgruppe gegebenenfalls abspaltet.

Die in der 7-Aminoverbindung der Formel VI vorhandene Carboxygruppe kann wahlweise geschützt sein, und zwar in der oben für die herzustellenden Ausgangsverbindungen der Formeln II, III und IV erläuterten Weise. Die Aminogruppe der Verbindung der Formel III kann z.B. durch eine Silylschutzgruppe, wie Trimethylsilyl, geschützt sein.

Als reaktionsfähige funktionelle Derivate von Säuren der Formel VII kommen z.B. Halogenide, d.h. Chloride, Bromide und Fluoride; Azide; Anhydride, insbesondere gemischte Anhydride mit stärkeren Säuren; reaktionsfähige Ester, z.B. N-Hydroxysuccinimidester, und Amide, z.B. Imidazolide, in Betracht.

Die Umsetzung der 7-Aminoverbindung der Formel VI mit der Säure der Formel VII oder einem reaktionsfähigen funktionellen Derivat davon kann in an sich bekannter Weise durchgeführt werden. So kann man z.B. eine freie Säure der Formel VII mit einem der erwähnten Ester entsprechend Formel VI mittels eines Carbodiimids, wie Dicyclohexylcarbodiimid, in einem inerten Lösungsmittel, wie Äthylacetat, Acetonitril, Dioxan, Chloroform, Methylenchlorid, Benzol oder Dimethylformamid, kondensieren und anschliessend die Estergruppe abspalten. Anstelle von Carbodiimiden lassen sich als Kondensationsmittel auch Oxazoliumsalze, z.B. N-Äthyl-5-phenyl-isoxazolium-3'-sulfonat, verwenden.

Nach einer anderen Ausführungsform setzt man ein Salz einer Säure der Formel VI, z.B. ein Trialkylammoniumsalz, wie das Triäthylammoniumsalz, mit einem reaktionsfähigen funktionellen Derivat einer Säure der Formel VII wie oben erwähnt in einem inerten Lösungsmittel, z.B. einem der oben genannten, um.

Nach einer weiteren Ausführungsform wird ein Säurehalogenid, vorzugsweise das Chlorid einer Säure der Formel VII mit dem Amin der Formel VI umgesetzt. Die Umsetzung erfolgt vorzugsweise in Gegenwart eines säurebindenden Mittels, z.B. in Gegenwart von wässrigem Alkali, vorzugsweise Natronlauge oder auch in Gegenwart eines Alkalimetallcarbonats, wie Kaliumcarbonat, oder in Gegenwart eines nieder-alkylierten Amins, wie Triäthylamin. Als Lösungsmittel wird vorzugsweise Wasser verwendet, ggf. im Gemisch mit einem inerten organischen Lösungsmittel, wie Tetrahydrofuran oder Dioxan. Es kann auch in einem aprotischen organischen Lösungsmittel, wie z.B. Dimethylformamid, Dimethylsulfoxid oder Hexamethylphosphorsäuretriamid, gearbeitet werden. Bei Verwendung von silylierten Ausgangsverbindungen der Formel VI wird in wasserfreiem Medium gearbeitet.

Die Umsetzung der 7-Aminoverbindung der Formel VI mit der Säure der Formel VII oder einem reaktionsfähigen funktionellen Derivat davon, kann zweckmässig bei Temperaturen zwischen etwa −40 °C und Zimmertemperatur, beispielsweise bei etwa 0–10 °C, erfolgen.

Ausgangsverbindungen der Formel III, worin $R^1$ die Gruppe $-CH_2SR^5$ darstellt und R nicht monohalogeniert ist, wie in Brom-, Chlor- oder Jodacetyl, können auch durch Thiolierung hergestellt werden, und zwar indem man eine Verbindung der allgemeinen Formel

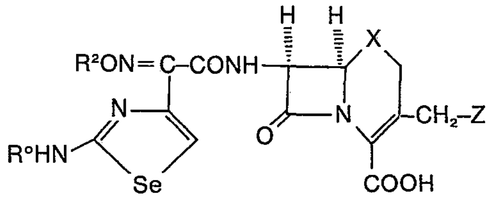

VIII

in der X und $R^2$ die oben gegebene Bedeutung haben, $R^0$ wie R ist, jedoch nicht monohalogeniert sein kann, Z eine austretende Gruppe darstellt und die Carboxygruppe durch Salzbildung mit einer anorganischen oder tertiären organischen Base geschützt sein kann,
in Gegenwart von Wasser mit einem Thiol der allgemeinen Formel

$$HS–R^5 \qquad V$$

in der $R^5$ die oben gegebene Bedeutung hat, umsetzt und eine allenfalls vorhandene Carboxyschutzgruppe gegebenenfalls abspaltet.

Als austretende Gruppe Z kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel VIII mit dem Thiol der Formel V kann in an sich bekannter Weise, z.B. bei einer Temperatur zwischen etwa 40 und 80 °C, zweckmässig bei etwa 60 °C, in Wasser oder in einer Pufferlösung mit einem pH von etwa 6 bis 7, vorzugsweise 6,5, durchgeführt werden. Die Carboxygruppe der erhaltenen Verbindung III kann erwünschtenfalls geschützt werden, z.B. durch Salzbildung oder Veresterung.

7-Aminoverbindungen der Formel VI, worin $R^1$ die Gruppe $-CH_2SR^5$ darstellt, können ausgehend von einer Verbindung der Formel

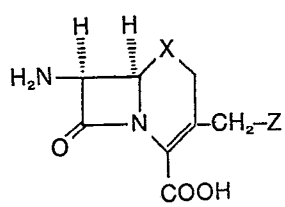

IX

in der X die oben gegebene Bedeutung hat, Z eine austretende Gruppe darstellt und die Carboxygruppe durch Salzbildung mit einer anorganischen oder tertiären organischen Base geschützt sein kann,
in Gegenwart von Wasser mit einem Thiol der Formel V hergestellt werden. Die Umsetzung kann unter den gleichen Bedingungen wie denjenigen erfolgen, wie sie für die Umsetzung der Ausgangsverbindungen VIII mit V beschrieben wurden. Andererseits können die Verbindungen der Formel VIII ausgehend von einer Verbindung der Formel IX und einer Säure der Formel VII oder einem reaktionsfähigen funktionellen Derivat davon unter den gleichen Bedingungen hergestellt werden,

wie sie für die Umsetzung der Verbindungen der Formeln VI und VII beschrieben wurden.

Die Carboxygruppe und/oder die Aminogruppe der erhaltenen Verbindung VI können erwünschtenfalls geschützt werden, z.B. durch Veresterung oder Salzbildung der Carboxygruppe oder durch Silylierung.

Die Säuren der Formel VII können z.B. durch Umsetzung einer Verbindung der allgemeinen Formel

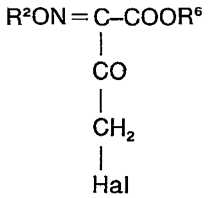

X

in der $R^2$ und Hal die oben gegebene Bedeutung haben und $R^6$ $C_{1-4}$-Alkyl, insbesondere Methyl oder Äthyl, darstellt,
mit Selenharnstoff hergestellt werden, in der gleichen Weise wie die obige Umsetzung der Ausgangsverbindungen der Formel II mit Selenharnstoff. Die so erhaltene Verbindung der Formel

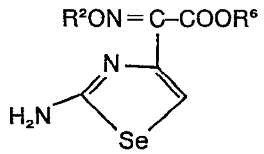

XI

worin $R^2$ und $R^6$ die oben gegebene Bedeutung haben,
wird anschliessend an der Aminogruppe geschützt, vorzugsweise durch Umsetzung mit dem entsprechenden Halogenid der Formel R–Hal, und die erhaltene Verbindung der Formel

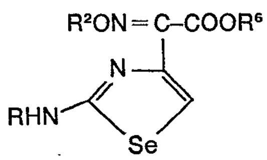

XII

worin R, $R^2$ und $R^6$ die oben gegebene Bedeutung haben,
zur Abspaltung der Estergruppe $R^6$ sauer oder alkalisch verseift. Die reaktionsfähigen Derivate der so erhaltenen Säuren der Formel VII werden in an sich bekannter Weise hergestellt.

Die Thiole der Formel V stehen im tautomeren Gleichgewicht mit den entsprechenden Thionen. Der Ätherrest von in 6-Stellung verätherten Thiolen (Thionen) wird im allgemeinen derart eingeführt, dass man ein S-geschütztes Thiol (z.B. durch Benzhydryl) mit dem die Äthergruppe enthaltenden Halogenid, vorzugsweise dem Jodid, in einem inerten organischen Lösungsmittel in Gegenwart eines säurebindenden Mittels, z.B. Kaliumcarbonat, umsetzt, vorzugsweise bei etwa 10°–50 °C, und die Schutzgruppe abspaltet (Benzhydryl kann mit Anisol und Trifluoressigsäure bei Raumtemperatur abgespalten werden).

Gemäss Verfahrensvariante b) des erfindungsgemässen Verfahrens wird die Aminoschutzgruppe R einer Ausgangsverbindung der Formel III abgespalten. Durch saure Hydrolyse abspaltbare Schutzgruppen werden vorzugsweise mit Hilfe einer niederen Alkancarbonsäure, die ggf. halogeniert sein kann, entfernt. Insbesondere verwendet man Ameisensäure oder Trifluoressigsäure. Die Temperatur ist in der Regel Raumtemperatur, obwohl auch leicht erhöhte bzw. leicht erniedrigte Temperatur angewendet werden kann, z.B. im Bereiche von etwa 0 °C bis +40 °C. Alkalisch abspaltbare Schutzgruppen werden im allgemeinen mit verdünnter wässriger Lauge bei 0 °C bis 30 °C hydrolysiert. Die Chloracetyl-, Bromacetyl- und Jodacetyl-Schutzgruppen können mittels Thioharnstoff in saurem, neutralem oder alkalischem Milieu bei etwa 0–30 °C abgespalten werden. Hydrogenolytische Abspaltung (z.B. Abspaltung von Benzyl) ist ungeeignet, da bei der Hydrogenolyse die Oximfunktion zur Aminogruppe reduziert wird.

Die allenfalls vorhandene Carboxyschutzgruppe kann in der gleichen Weise wie oben für die Verfahrensvariante a) beschrieben abgespalten werden. Die Carboxyschutzgruppe kann in der gleichen Weise auch vor der Abspaltung der Schutzgruppe R abgespalten werden.

Die für die Verfahrensvariante c) des erfindungsgemässen Verfahrens einsetzbaren Ausgangsverbindungen der allgemeinen Formel IV können durch Abspaltung der Aminoschutzgruppe R der oben beschriebenen Verbindungen der Formel VIII erhalten werden. Diese Abspaltung kann in der gleichen Weise durchgeführt werden wie die oben beschriebene Abspaltung der Aminoschutzgruppen R der Ausgangsverbindungen der Formel III.

Als austretende Gruppe Z einer Verbindung der Formel IV kommen beispielsweise Halogene, z.B. Chlor, Brom oder Jod, Acyloxyreste, z.B. niedere Alkanoyloxyreste, wie Acetoxy, niedere Alkyl- oder Arylsulfonyloxyreste, wie Mesyloxy oder Tosyloxy, oder der Azidorest in Frage.

Die Umsetzung der Verbindung der Formel IV mit dem Thiol der Formel V kann in an sich bekannter Weise, z.B. bei einer Temperatur zwischen etwa 40 und 80 °C, zweckmässig bei etwa 60 °C, in Wasser oder in einer Pufferlösung mit einem pH von etwa 6 bis 7, vorzugsweise 6,5, durchgeführt werden. Die allenfalls vorhandene Carboxyschutzgruppe kann in der gleichen Weise wie oben für die Verfahrensvariante a) beschrieben abgespalten werden.

Die Variante d) des erfindungsgemässen Verfahrens, die Oxidation von Verbindungen der Formel I, worin X Schwefel oder die Gruppe –SO– darstellt bzw. eines Salzes davon, erfolgt durch Behandlung mit einem organischen oder anorganischen Oxidationsmittel. Als Oxidationsmittel dienen verschiedene, Sauerstoff leicht abgebende Verbindungen, wie z.B. organische Peroxide, z.B. monosubstituierte organische Peroxide, wie $C_1$–$C_4$ Alkyl- oder Alkanoylhydroperoxide, wie t-Butylhydroperoxid, Perameisensäure, Peressigsäure; sowie phenylsubstituierte Derivate dieser

Hydroperoxide, wie Cumolhydroperoxid, Perbenzoesäure. Der Phenylsubstituent kann gegebenenfalls eine weitere niedere Gruppe, z.B. $C_1$–$C_4$ Alkyl oder Alkoxy, Halogen oder Carboxygruppe tragen, z.B. 4-Methylperbenzoesäure, 4-Methoxyperbenzoesäure, 3-Chlorperbenzoesäure, Monoperphthalsäure. Als Oxidationsmittel können auch verschiedene anorganische Oxidationsmittel verwendet werden, z.B. Wasserstoffperoxid; Ozon; Permanganate, wie Kalium- oder Natriumpermanganat; Hypochlorite, wie Natrium-, Kalium- oder Ammoniumhypochlorit; Peroxymono- und Peroxydischwefelsäure. Bevorzugt ist die Verwendung von 3-Chlorperbenzoesäure. Die Oxidation erfolgt mit Vorteil in einem inerten Lösungsmittel, z.B. in einem aprotischen inerten Lösungsmittel, wie Tetrahydrofuran, Dioxan, Methylenchlorid, Chloroform, Äthylacetat oder Aceton; oder in einem protischen Lösungsmittel, wie Wasser, einem niederen Alkanol, z.B. Methanol, Äthanol, oder einer niederen, ggf. halogenierten Alkancarbonsäure, z.B. Ameisensäure, Essigsäure, Trifluoressigsäure. Die Reaktionstemperatur bewegt sich vornehmlich im Bereiche von $-20\,°C$ bis $+50\,°C$.

Bei Verwendung von äquimolaren Mengen Oxidationsmittel bzw. leichtem Überschuss im Verhältnis zur Ausgangsverbindung der Formel I, worin X Schwefel darstellt bzw. einem Salz davon, erhält man vornehmlich das entsprechende Sulfoxid der Formel I, worin X die Gruppe –SO– darstellt. Erhöht man die Menge des Oxidationsmittels auf das doppelte stöchiometrische Verhältnis oder mehr, bildet sich das entsprechende Sulfon der Formel I, worin X die Gruppe $-SO_2-$ darstellt. Es ist ebenfalls möglich, das Sulfon der Formel I aus dem entsprechenden Sulfoxid durch Behandlung mit dem Oxidationsmittel in äquimolarer oder grösserer Menge zu erhalten. Die Verfahrensbedingungen sind im wesentlichen die gleichen wie bei der Herstellung des Sulfoxids.

Zur Herstellung der leicht hydrolysierbaren (Mono- oder Di-) Ester der Carbonsäuren der Formel I gemäss Variante e) wird die Carbonsäure vorzugsweise mit dem entsprechenden, die Estergruppe enthaltenden Halogenid, bevorzugt mit dem Jodid, umgesetzt. Die Reaktion kann mit Hilfe einer Base, z.B. einem Alkalimetallhydroxid oder -carbonat oder einem organischen Amin, wie Triäthylamin beschleunigt werden. Bei Vorhandensein eines Rests $-CH_2SR^5$ als Bedeutung für $R^1$ mit einem hydroxysubstituierten Heterocyclus werden die Hydroxygruppen unter Bildung von entsprechenden, leicht hydrolysierbaren Äthern veräthert. Vorzugsweise verwendet man dabei einen Überschuss an dem entsprechenden Halogenid. Die Veresterungs-/Verätherungsreaktion wird vorzugsweise in einem inerten organischen Lösungsmittel durchgeführt, wie Dimethylacetamid, Hexamethylphosphorsäuretriamid, Dimethylsulfoxid oder, vorzugsweise, Dimethylformamid. Die Temperatur liegt vorzugsweise im Bereiche von etwa 0–40 °C.

Die Herstellung der Salze und Hydrate der Verbindungen der Formel I bzw. der Hydrate dieser Salze kann in an sich bekannter Weise erfolgen, z.B. durch Umsetzung einer Carbonsäure der Formel I mit einer äquivalenten Menge der gewünschten Base, zweckmässig in einem Lösungsmittel, wie Wasser oder in einem organischen Lösungsmittel, wie Äthanol, Methanol, Aceton und anderen mehr. Bei Verwendung eines zweiten bzw. dritten Äquivalents an Base erfolgt Salzbildung auch an einem allenfalls vorhandenen hydroxysubstituierten Heterocyclus in Stellung 3 bzw. an der Carboxygruppe einer Carboxy-niederalkylgruppe $R^2$, wobei ein Di- bzw. Trisalz entsteht. Die Temperatur der Salzbildung ist nicht kritisch. Sie liegt im allgemeinen bei Raumtemperatur, kann aber auch leicht darüber oder darunter, etwa im Bereich von $0\,°C$ bis $+50\,°C$, sein.

Die Herstellung der Hydrate erfolgt zumeist automatisch im Zuge des Herstellungsverfahrens oder als Folge hygroskopischer Eigenschaften eines zunächst wasserfreien Produktes. Zur gezielten Herstellung eines Hydrats kann ein ganz oder teilweise wasserfreies Produkt (Carbonsäure der Formel I bzw. Ester, Äther oder Salz davon) einer feuchten Atmosphäre, z.B. bei etwa $+10\,°C$ bis $+40\,°C$, ausgesetzt werden.

Ein allenfalls erhaltenes syn/anti-Gemisch einer Verbindung der Formel I kann in die entsprechenden syn- und anti-Formen in üblicher Weise aufgetrennt werden, beispielsweise durch Umkristallisieren oder durch chromatographische Methoden unter Verwendung eines geeigneten Lösungsmittels bzw. Lösungsmittelgemisches.

Die Verbindungen der Formel I und III sowie die entsprechenden leicht hydrolysierbaren Ester, Äther und Salze bzw. die Hydrate dieser Produkte sind antibiotisch, insbesondere bakterizid wirksam. Sie besitzen ein breites Wirkungsspektrum gegen Gram-positive und Gram-negative Mikroorganismen, einschliesslich β-Lactamase-bildende Staphylokokken, Streptokokken und verschiedene β-Lactamase-bildende Gram-negative Bakterien, wie z.B. Pseudomonas aeruginosa, Escherichia coli, Serratia marcescens, Proteus- und Klebsiella-Spezies.

Die Verbindungen der Formel I und III sowie die entsprechenden leicht hydrolysierbaren Ester, Äther und Salze bzw. die Hydrate dieser Produkte können zur Behandlung und Prophylaxe von Infektionskrankheiten verwendet werden. Für den Erwachsenen kommt eine Tagesdosis von etwa 0,1 g bis etwa 2 g in Betracht. Die parenterale Verabreichung der erfindungsgemässen Verbindungen ist besonders bevorzugt.

Zum Nachweis der antimikrobiellen Wirksamkeit der erwähnten Produkte wurden folgende repräsentative Vertreter getestet:

Produkt A:
(6R,7R)-7-[2-(2-Amino-4-selenazolyl-2-[(Z)-methoxyimino]-acetamido]-3-[[(1-methyl-1H-tetrazol-5-yl)-thio]-methyl]-8-oxo-5-thia-1-azabicyclo[4.2.0]oct-2-en-2-carbonsäure-Natriumsalz

Produkt B:
(6R,7R)-7-[2-(2-Amino-4-selenazolyl-2-[(Z)-methoxyimino]-acetamido]-3-[[(5-methyl-1,3,4-thiadiazol-2-yl)thio]methyl]-8-oxo-5-thia-1-azabicyclo[4.2.0]oct-2-en-2-carbonsäure-Natriumsalz

Produkt C:
(6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)thio]-methyl]-8-oxo-5-thia-1-azabicyclo[4.2.0]oct-2-en-2-carbonsäure-Dinatriumsalz

Produkt D:
(6R,7R)-3-(Acetoxymethyl)-7-[2-(2-amino-4-selenazolyl)-2-[(Z)-methoxyimino]-acetamido]-8-oxo-5-thia-1-azabicyclo[4.2.0]oct-2-en-2-carbonsäure-Natriumsalz

Produkt E:
(6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure-5-oxid-Dinatriumsalz

Produkt F:
(6R,7R)-7-[2-(2-Amino-4-selenazolyl)-2-[(Z)-methoxyimino]-acetamido]-3-methyl-3-oxo-5-thia-1-azabicyclo[4.2.0]oct-2-en-2-carbonsäure-Natriumsalz

Produkt G:
(6R,7R)-7-[2-(2-Amino-4-selenazolyl)-2-[(Z)-methoxyimino]-acetamido]-8-oxo-3-[[(1,4,5,6-tetrahydro-4-methyl-5,6-dioxo-as-triazin-3-yl)thio]methyl]-5-thia-1-azabicyclo[4.2.0]oct-2-en-2-carbonsäure-Natriumsalz

Produkt H:
Methylen (6R,7R)-7-[2-(2-Amino-4-selenazolyl)-2-[(Z)-methoxyimino]acetamido]-3-methyl-8-oxo-5-thia-1-azabicyclo[4.2.0]oct-2-en-2-carboxylat-pivalat

Produkt I:
(6R,7R)-3-(Acetoxymethyl)-7-[(Z)-2-(2-amino-4-selenazolyl)-2-[(1-carboxy-1-methylmethoxy)imino]acetamido]-8-oxo-5-thia-1-azabicyclo[4.2.0]oct-2-en-2-carbonsäure-Dinatriumsalz

Produkt K:
1-[[(6R,7R)-7-[(Z)-2-(2-Amino-4-selenazolyl)-2-[(1-carboxy-1-methyläthoxy)imino]acetamido]-2-carboxy-8-oxo-5-thia-1-azabicyclo-[4.2.0]oct-2-en-3-yl]methyl]pyridinium-hydroxid-Natriumsalz (inneres Salz)

Produkt L:
(6R,7R)-3-(Acetoxymethyl)-7-[-2-(2-amino-4-selenazolyl)-2-[(Z)-hydroxyimino]-acetamido]-8-oxo-5-thia-1-azabicyclo[4.2.0]oct-2-en-2-carbonsäure-Natriumsalz

Produkt M:
(6R,7R)-7-[2-[2-Amino-4-selenazolyl]-2-[(Z)-hydroxyimino]-acetamido]-3-methyl-8-oxo-5-thia-1-azabicyclo[4.2.0]oct-2-en-2-carbonsäure-Natriumsalz.

Aktivität in vitro: Mindesthemmkonzentration (µg/ml)

| Erreger | A | B | C | D | E | F | G | I | K | L | M |
|---|---|---|---|---|---|---|---|---|---|---|---|
| Escherichia coli | | | | | | | | | | | |
| Stamm 1 | 0,1 | 0,2 | 0,1 | 0,1 | 0,1 | 0,8 | 0,05 | 1,6 | 0,4 | ≤0,025 | 0,8 |
| Stamm 2 | 0,025 | 0,006 | 0,05 | 0,025 | 0,025 | 0,05 | 0,012 | 0,2 | 0,1 | ≤0,025 | 0,4 |
| Stamm 3 | 0,4 | 0,8 | 0,8 | | 0,8 | 12,5 | 0,2 | 0,8 | 0,8 | 1,6 | 50 |
| Stamm 4 | 0,2 | 0,2 | 0,4 | | 0,05 | 100 | 0,2 | – | 25 | 6,3 | 200 |
| Stamm 5 | 0,4 | 0,4 | 0,2 | | 0,4 | 3,1 | 0,2 | 0,4 | 0,4 | 0,1 | 3,1 |
| Proteus mirabilis | | | | | | | | | | | |
| Stamm 1 | 0,10 | 0,19 | 0,012 | 0,025 | | | | | | | |
| Stamm 2 | 0,10 | 0,19 | 0,025 | 0,05 | | | | | | | |
| Stamm 3 | 0,8 | 0,2 | 1,6 | | 0,05 | 0,1 | 0,4 | 0,1 | 0,4 | ≤0,025 | 0,8 |
| Stamm 4 | 0,05 | 0,4 | 0,012 | | 0,4 | 0,8 | 0,025 | 0,1 | 0,1 | 0,05 | 6,3 |
| Stamm 5 | 0,05 | 0,4 | 0,012 | | 0,1 | 0,4 | 0,025 | 0,2 | 0,05 | 0,05 | 1,6 |
| Proteus morganii | 0,1 | 0,05 | 0,05 | 0,39 | 0,1 | 50 | 0,05 | 0,8 | 0,2 | 0,4 | 100 |
| Proteus rettgeri | 0,2 | 0,2 | 0,1 | | 0,05 | 0,1 | 0,05 | 0,1 | – | – | 0,2 |
| Proteus inconstans | 0,025 | 0,025 | 0,006 | | 0,025 | ≤0,025 | 0,006 | 0,012 | 0,05 | ≤0,025 | 0,05 |
| Proteus vulgaris | 0,78 | 0,78 | 1,6 | 0,78 | | | | | | | |
| Klebsiella pneumoniae | | | | | | | | | | | |
| Stamm 1 | 0,2 | 0,4 | 0,1 | 0,2 | 0,1 | 0,2 | 0,1 | 0,8 | 0,8 | ≤0,025 | 0,8 |
| Stamm 2 | 0,05 | 0,1 | 0,05 | 0,025 | 0,05 | 0,05 | 0,025 | 0,4 | 0,2 | ≤0,025 | 0,4 |
| Stamm 3 | 0,1 | 0,2 | 0,1 | | 0,1 | 0,05 | 0,05 | 0,8 | 0,8 | ≤0,025 | 0,4 |

| Erreger | A | B | C | D | E | F | G | I | K | L | M |
|---|---|---|---|---|---|---|---|---|---|---|---|
| Serratia marcescens | | | | | | | | | | | |
| Stamm 1 | 0,4 | 0,8 | 0,4 | 0,8 | 0,8 | 3,1 | 0,2 | 1,6 | 0,8 | 0,8 | 25 |
| Stamm 2 | 0,8 | 1,6 | 1,6 | 0,8 | 0,8 | 6,3 | 0,4 | 3,1 | 0,8 | 1,6 | 100 |
| Stamm 3 | 0,1 | 0,2 | 0,1 | | 0,2 | 0,4 | 0,1 | 0,4 | 0,1 | 0,1 | 6,3 |
| Enterobacter cloacae | | | | | | | | | | | |
| Stamm 1 | >100 | >100 | >100 | >100 | 1,6 | >200 | >100 | >100 | >100 | 200 | >200 |
| Stamm 2 | 0,8 | 1,6 | 3,1 | 3,1 | 0,2 | 25 | 0,8 | 6,3 | 1,6 | 6,3 | >200 |
| Stamm 3 | 50 | 50 | >100 | | 1,6 | >200 | 50 | >100 | 100 | 25 | >200 |
| Enterobacter aerogenes | | | | | | | | | | | |
| Stamm 1 | 0,1 | 0,1 | 0,1 | | 0,1 | 0,2 | 0,1 | 0,4 | 0,4 | ≤0,025 | 0,4 |
| Stamm 2 | 0,1 | 0,1 | 0,1 | | 0,05 | 0,2 | 0,1 | 0,2 | 0,2 | 0,4 | 1,6 |
| Citrobacter freundii | | | | | | | | | | | |
| Stamm 1 | 1,6 | 1,6 | 12,5 | 6,3 | 0,1 | 200 | 1,6 | 100 | 12,5 | 3,1 | 100 |
| Stamm 2 | 0,05 | 0,2 | 0,1 | | 0,1 | 1,6 | 0,1 | 1,6 | 0,4 | 0,025 | 1,6 |
| Pseudomonas aeruginosa | | | | | | | | | | | |
| Stamm 1 | 25 | 50 | 3,1 | 12,5 | 100 | >200 | 50 | | | 50 | >400 |
| Stamm 2 | 25 | 25 | 6,3 | 12,5 | 100 | >200 | 50 | | | | |
| Stamm 3 | 6,3 | 6,3 | 1,6 | 3,1 | 50 | 200 | 25 | 1,6 | 1,6 | 12,5 | >400 |
| Staphylococcus aureus | | | | | | | | | | | |
| Stamm 1 | 0,8 | 0,8 | 3,1 | 0,8 | >12 | 50 | 0,4 | 50 | 12,5 | 0,2 | 3,1 |
| Stamm 2 | 1,6 | 1,6 | 6,3 | 3,1 | >12 | 100 | 1,6 | 100 | 50 | 0,4 | 6,3 |
| Stamm 3 | 0,8 | 0,8 | 3,1 | | >12 | 50 | 0,8 | 50 | 12,5 | 0,4 | 3,1 |
| Stamm 4 | 0,8 | 1,6 | 6,3 | | >12 | 100 | 0,8 | 100 | 12,5 | 0,4 | 6,3 |
| Staphylococcus epidermidis | 0,8 | 0,8 | 3,1 | | >12 | 100 | 0,8 | 50 | 12,5 | 0,4 | 3,1 |
| Streptococcus faecalis | | | | | | | | | | | |
| Stamm 1 | 100 | 100 | >100 | | >12 | >200 | 25 | >100 | >100 | 25 | 200 |
| Stamm 2 | >100 | 100 | >100 | | >12 | >200 | 50 | >100 | >100 | 25 | >200 |
| Streptococcus pyogenes | | | | | | | | | | | |
| Stamm 1 | 0,025 | 0,025 | 0,05 | 0,05 | 3,1 | 3,1 | 0,05 | 0,2 | 0,4 | 0,05 | 1,6 |
| Stamm 2 | ≤0,012 | ≤0,012 | ≤0,012 | | 0,2 | 0,05 | – | 0,05 | 0,1 | ≥0,025 | 0,1 |
| Streptococcus pneumoniae | 0,025 | 0,025 | 0,05 | | 3,1 | 12,5 | 0,1 | 0,2 | 0,1 | 0,1 | 6,3 |
| Streptococcus viridans | 0,8 | 0,8 | 3,1 | | >12 | 12,5 | 0,8 | 6,3 | 3,1 | 0,8 | 25 |

Aktivität in vivo

Gruppen von 5 Mäusen werden mit einer Suspension verschiedener Krankheitserreger intraperitoneal infiziert. Falls nicht anders in untenstehender Tabelle angegeben, wurde die Prüfsubstanz zweimal, d.h. 1 Stunde und 3 Stunden nach der Infektion, in physiologischer Kochsalzlösung subcutan appliziert. Die Zahl der überlebenden Tiere wird am 4. Tag bestimmt. Es werden verschiedene Dosierungen appliziert, und es wird nach der Probitmethode diejenige Dosis bestimmt, bei der 50% der Versuchstiere überleben ($CD_{50}$, mg/kg).

Aktivität in vivo: $CD_{50}$, mg/kg

| Erreger | A | B | C | D | E | F | G | H | I | K | L | M |
|---|---|---|---|---|---|---|---|---|---|---|---|---|
| Escherichia coli | 0,006 | 0,006 | <0,003 | <0,01 | 0,017 | 0,06 | < 0,003 | 0,11 | 0,067 | | | 1,0 |
| Pseudomonas aeruginosa* | 39 | 134 | 14 | 35 | 75 | >200 | >200 | >100 | 23 | 1,1 | | |
| Klebsiella pneumoniae | | | 0,23 | | | | | 0,44 | | | | |
| Neisseria meningitidis** | | | <0,05 | | <0,1 | | | | | | | |
| Haemophilus influenza | 1,5 | 0,27 | <0,05 | | 1,8 | 4 | 0,25 | | | | | |
| Enterobacter cloacae | 8,4 | >12 | 17 | | 0,06 | >25 | 9,2 | | >50 | | | |
| Serratia marcescens | | | | | | | | | | | | |
| Stamm 1 | 0,13 | 0,52 | 0,07 | 0,14 | 0,03 | 0,59 | 0,2 | | 1,8 | 0,11 | | 3,5 |
| Stamm 2 | | | | | | 5,1 | | | | | | >50 |
| Staphylococcus aureus | | | | | | | | >12 | | | | 2,7 |
| Streptococcus pyogenes | 0,05 | 0,05 | 0,04 | 0,05 | 0,13 | <0,05 | 0,02 | 0,13 | 2,7 | 0,28 | 0,08 | 0,23 |

* Dreimal subcutan appliziert: 1 Stunde, 3 Stunden und 5 Stunden nach der Infektion.
** Zweimal subcutan appliziert: 1 Stunde und 4 Stunden nach der Infektion.

Toxizität, $LD_{50}$, mg/kg

| Prüfsubstanz | Verabreichungsmodus s.c. | p.o. |
|---|---|---|
| A (Endprodukt von Beispiel 6) | >4000 | >5000 |
| B (Endprodukt von Beispiel 5) | >4000 | >5000 |
| C (Endprodukt von Beispiel 1) | >4000 | >5000 |
| D (Endprodukt von Beispiel 8) | >4000 | >5000 |
| E (Endprodukt von Beispiel 2) | >4000 | >5000 |
| F (Endprodukt von Beispiel 3) | >4000 | >5000 |
| G (Endprodukt von Beispiel 7) | >4000 | >5000 |
| H (Endprodukt von Beispiel 4) | | >5000 |

Die erfindungsgemässen Produkte können als Heilmittel z.B. in Form pharmazeutischer Präparate Verwendung finden, welche sie oder ihre Salze in Mischung mit einem für die enterale oder parenterale Applikation geeigneten pharmazeutischen, organischen oder anorganischen inerten Trägermaterial, wie z.B. Wasser, Gelatine, Gummi arabicum, Milchzucker, Stärke, Magnesiumstearat, Talk, pflanzliche Öle, Polyalkylenglykole, Vaseline usw. enthalten. Die pharmazeutischen Präparate können in fester Form, z.B. als Tabletten, Dragées, Suppositorien, Kapseln; oder in flüssiger Form, z.B. als Lösungen, Suspensionen oder Emulsionen, vorliegen. Gegebenenfalls sind sie sterilisiert und bzw. oder enthalten Hilfsstoffe, wie Konservierungs-, Stabilisierungs-, Netz- oder Emulgiermittel, Salze zur Veränderung des osmotischen Druckes, Anaesthetica oder Puffer. Sie können auch noch andere therapeutische wertvolle Stoffe enthalten. Die Verbindungen der Formel I und ihre Salze bzw. Hydrate kommen vorzugsweise für die parenterale Applikation in Betracht und werden zu diesem Zweck bevorzugt als Lyophilisate oder Trockenpulver zur Verdünnung mit üblichen Agenzien wie Wasser oder isotonische Kochsalzlösung, zubereitet. Die leicht hydrolysierbaren Ester bzw. Äther der Verbindungen der Formel I und ihre Salze bzw. Hydrate kommen auch für die enterale (z.B. orale) Verabreichung in Betracht.

Cephalosporinderivate mit Schwefel anstelle des in den erfindungsgemässen Endprodukten vorhandenen Selenatoms sind bekannt, z.B. die Handelsprodukte Ceftriaxon, Cefmenoxim, Ceftazidim und Cefotaxim.

Beispiel 1

Herstellung des Dinatriumsalzes der
(6R,7R)-7- [2-(2-Amino-4-selenazolyl)-2-[(Z)-methoxyimino]-acetamido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure.

11,55 g (6R,7R)-7-[4-Brom-2-[(Z)-methoxyimino]-3-oxo-butyramido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure werden in 160 ml Äthanol gelöst und bei 25 °C mit 2,7 g Selenharnstoff versetzt. Das Gemisch wird 1½ Stunden bei 25 °C unter Stickstoff-Begasung und Lichtausschluss gerührt, wobei zunächst harziges Material ausfällt und dann Kristallisation eintritt. Das auskristallisierte Hydrobromid wird abgenutscht, mit Äthanol und tiefsiedendem Petroläther gewaschen und im Vakuum bei 40 °C getrocknet. Man erhält rötliches Hydrobromid der Titelsubstanz, welches, zwecks Überführung in das Dinatriumsalz, zunächst zusammen mit 4,5 g Natriumacetat-Trihydrat in einem Gemisch von 35 ml Wasser und 35 ml Aceton gelöst wird. Dieser Lösung werden 35 ml Aceton bis zur Trübung zugegeben. Wenig ungelöstes, rötliches Selen wird mittels Faltenfilter abfiltriert. Dem orange-gelben Filtrat werden weitere 15 ml Aceton hinzugefügt. Das dabei abgeschiedene, ölig-harzige Material wird mittels Faltenfilter abgetrennt und verworfen. Durch Reiben mit einem Glasstab wird die Kristallisation des Dinatriumsalzes aus dem gelben Filtrat induziert. Man lässt 1 Stunde kristallisieren, und anschliessend werden dem Kristallisationsgemisch noch 50 ml Aceton bis zur vollständigen Kristallisation unter Rühren während ca. 1 Stunde zugetropft. Das Kristallisat wird abgenutscht, mit 50 ml Aceton-Wasser (85 : 15), Aceton und tiefsiedendem Petroläther gewaschen und über Nacht im Vakuum bei 25 °C getrocknet. Man erhält hellbeige, voluminöse Titelsubstanz mit $[\alpha]_D^{25} = -139$ °C (c = 0,5 in Wasser). Das Kernresonanzspektrum und die Mikroanalyse entsprechen der angegebenen Struktur. Die Substanz enthält 3,5 Mol Kristallwasser.

Die als Ausgangsverbindung eingesetzte
(6R,7R)-7-[4-Brom-2-[(Z)-methoxyimino]-3-oxo-butyramido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure
kann wie folgt hergestellt werden:

592,1 g Acetessigsäure-tert.-butylester werden in 560 ml Eisessig gelöst. Zu dieser Lösung wird bei 5–10 °C während 2½ Stunden eine Lösung von 290,6 g Natriumnitrit in 655 ml Wasser getropft. Die entstandene gelbe Suspension wird 30 Minuten bei 20 °C gerührt, mit 940 ml Wasser versetzt und weitere 2 Stunden gerührt. Das Gemisch wird mit 900 ml Wasser und 900 g Eis versetzt und im

Ausrührgefäss dreimal mit je 1 l Äthylacetat extrahiert. Die vereinigten Äthylacetatextrakte werden dreimal mit je 1 l Wasser gewaschen, dann mit 5 l Wasser versetzt und mit Natriumhydrogencarbonat der pH-Wert auf 6,8 gestellt. Nach dem Abtrennen der wässrigen Phase wird noch einmal mit Wasser gewaschen. Danach wird die Äthylacetatlösung über Natriumsulfat getrocknet und am Vakuum bei 40 °C eingedampft. Man erhält (Z)-2-Hydroxyimino-3-oxo-buttersäure-tert.-butylester als gelbes Öl, das noch 9 Stunden am Hochvakuum bei 40 °C getrocknet wird.

626,65 g (Z)-2-Hydroxyimino-2-oxo-buttersäure-tert.-butylester werden in 2,86 l Aceton gelöst. Die Lösung wird auf 5 °C abgekühlt und portionenweise mit 703,5 g Kaliumcarbonat versetzt. Zur gelben Suspension werden dann ohne Kühlung während 1 Stunde 322 ml Dimethylsulfat hinzugetropft, wobei die Temperatur des Gemisches nicht über 25 °C steigen soll. Die hellbeige Suspension wird bei 20–25 °C ca. 4 Stunden gerührt, bis dünnschichtchromatographisch kein Ausgangsmaterial mehr nachgewiesen wird. Danach wird das Gemisch auf 7 l Wasser gegossen und dreimal mit je 1 l Äthylacetat extrahiert. Die vereinigten Äthylacetatextrakte werden dreimal mit je 1 l Wasser gewaschen, über Natriumsulfat getrocknet und am Vakuum bei 40 °C eingedampft. Das verbleibende, gelbe Öl wird noch 6 Stunden am Hochvakuum bei 40 °C getrocknet und anschliessend destilliert. Man erhält (Z)-2-Methoxyimino-3-oxo-buttersäure-tert.-butylester als gelbes Öl mit einem Siedepunkt von 57 °C bei 0,02 mmHg.

86 g (Z)-2-Methoxyimino-3-oxo-buttersäure-tert.-butylester werden in 400 ml Trifluoressigsäure gelöst. Die Lösung wird 1 Stunde bei 25 °C stehen gelassen, danach am Vakuum bei 35 °C eingedampft. Der ölige Rückstand wird aus Äther/Petroläther kristallisiert. Man erhält gelbliche, wasserlösliche (Z)-2-Methoxyimino-3-oxo-buttersäure vom Schmelzpunkt 80–85 °C.

145 g (Z)-2-Methoxyimino-3-oxo-buttersäure werden in 1000 ml alkohol- und wasserfreiem Dichlormethan gelöst. Zu dieser Lösung werden 10 ml 30%ige Bromwasserstoffsäure in Eisessig gegeben. Dann wird während ca. 2 Stunden eine Lösung von 37,5 ml Brom in 112,5 ml Dichlormethan zugetropft, wobei die Temperatur des Reaktionsgemisches mittels leichter Kühlung auf 20–25 °C gehalten wird. Nun wird zur Entfernung von HBr aus dem Reaktionsgemisch heftig Stickstoff durchgeblasen. Anschliessend werden nacheinander 250 g Eis, 250 ml Wasser und 2 l Äther zugegeben. Die wässrige Phase wird abgetrennt und verworfen. Die organische Phase wird mit 250 ml Wasser und 250 ml gesättigter Natriumchloridlösung gewaschen, mit Natriumsulfat getrocknet und im Vakuum eingedampft. Es verbleibt ein braunes Öl, das aus Tetrachlorkohlenstoff kristallisiert wird. Man erhält praktisch farblose (Z)-4-Brom-2-methoxyimino-3-oxo-buttersäure.

37,1 g (7R)-7-Amino-3-desacetoxy-3-[(2,5-dihydro-6-hydroxy-2-methyl-5-oxo-as-triazin-3-yl)thio]cephalosporansäure werden in 800 ml Essigester suspendiert und mit 100 ml N,O-Bis-(trimethylsilyl)-acetamid versetzt. Das Gemisch wird unter Feuchtigkeitsausschluss 30 Minuten bei 25 °C gerührt, wobei eine hellgelbe Lösung entsteht. Dieser auf −10 °C gekühlten Lösung wird eine Lösung von

(Z)-4-Brom-2-methoxyimino-3-oxo-buttersäurechlorid,

welches aus 22,4 g

(Z)-4-Brom-2-methoxyimino-3-oxo-buttersäure

in 300 ml alkohol- und wasserfreiem Dichlormethan und 20,8 g Phosphorpentachlorid bei 8–10 °C hergestellt worden war, während 30 Minuten bei −10 bis 0 °C zugetropft. Das Reaktionsgemisch wird 30 Minuten bei 0–5 °C und 1 Stunde bei 25 °C gerührt. Es werden 1 l Essigester und 500 ml Wasser unter Rühren hinzugefügt. Die wässrige Phase und die harzige Zwischenschicht werden verworfen. Die organische Phase wird sechsmal mit je 500 ml Wasser gewaschen, mit Natriumsulfat getrocknet und am Vakuum bei 40 °C auf ein Volumen von 200 ml eingeengt. Dieses orange gefärbte Konzentrat wird unter Rühren zu 1,8 l Äther getropft, wobei das Reaktionsprodukt amorph ausfällt. Dieses wird abgenutscht, nacheinander mit 1 l Äther und 1 l tiefsiedendem Petroläther gewaschen und über Nacht im Vakuum bei 35 °C getrocknet. Man erhält

(6R,7R)-7-[4-Brom-2-[(Z)-methoxyimino]-3-oxobutyramido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure

als beiges, amorphes Produkt mit $[\alpha]_D^{25}$ = −223,1° (c = 1 in Methanol).

Beispiel 2

Herstellung des Dinatriumsalzes von (6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure-5-oxid:

5,93 g (6R,7R)-7-[4-Brom-2-[(Z)-methoxy-imino]-3-oxobutyramido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure-5-oxid werden in einem Gemisch von 30 ml Wasser und 30 ml Aceton suspendiert und mit 4,5 g Natriumacetat-trihydrat sowie 1,35 g Selenharnstoff versetzt. Das Gemisch wird 30 Minuten bei 25 °C gerührt. Etwas ungelöstes, rötliches Material wird mittels Faltenfilter abgetrennt. Das orange-gelbe Filtrat wird mit 15 ml Aceton bis zur Trübung versetzt, wobei das Reaktionsprodukt sofort kristallisiert. Das Gemisch wird noch 15 Minuten gerührt, dann wird das Kristallisat abgenutscht, mit 50 ml Aceton-Wasser (85 : 15), Aceton und tiefsiedendem Petroläther gewaschen und im Vakuum bei 25 °C getrocknet. Man erhält beige, rohe Titelsubstanz, welche, zwecks Umkristallisation, zunächst in 30 ml Wasser gelöst wird. Wenig ungelöstes

Selen wird mittels Faltenfilter entfernt. Das Filtrat wird mit Aceton bis zur Trübung versetzt, wobei das Dinatriumsalz sofort auskristallisiert. Das Gemisch wird noch 15 Minuten gerührt. Das Dinatriumsalz wird abgenutscht, mit 50 ml Aceton-Wasser (85 : 15), Aceton und tiefsiedendem Petroläther gewaschen und über Nacht im Hochvakuum bei 40–45 °C getrocknet. Danach wird die Substanz, zwecks Erzielung eines konstanten Wassergehaltes, 1 Stunde an der Luft äquilibriert. Man erhält hellbeige reine kristalline Titelsubstanz mit $[\alpha]_D^{25} = -117{,}5°$ (c = 0,8 in Wasser). Das Kernresonanzspektrum und die Mikroanalyse entsprechen der angegebenen Struktur. Die Substanz enthält 6 Mol Wasser.

Das oben als Ausgangsmaterial verwendete
(6R,7R)-7-[4-Brom-2-[(Z)-methoxyimino]-3-oxobutyramido]-3-[[(2,5-dihydro-6-hydroxy-2-methyl-5-oxo-as-triazin-3-yl)thio]-methyl]-8-oxo-5-thia-1-azabicyclo[4.2.0]oct-2-en-2-carbonsäure-5-oxid
kann wie folgt hergestellt werden:

17,31 g (6R,7R)-7-[4-Brom-2-[(Z)-methoxyimino]-3-oxo-butyramido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure
werden in 170 ml Äthanol gelöst und während 45 Minuten portionenweise mit 6,1 g 3-Chlorperbenzoesäure (85%ig) versetzt. Das Reaktionsprodukt fällt aus; die Reaktion ist leicht exotherm; man beobachtet einen Temperaturanstieg von 25 °C auf 30 °C. Das Gemisch wird nach erfolgter Zugabe des Oxidationsmittels noch 15 Minuten bei ca. 25 °C gerührt. Die Substanz wird abgenutscht, mit wenig Äthanol, Äther und tiefsiedendem Petroläther gewaschen und im Vakuum bei 40 °C getrocknet. Man erhält somit eine Fraktion I des gewünschten Produktes. Die Mutterlauge wird im Vakuum bei 40 °C stark eingeengt. Das dabei auskristallisierte Material wird abgenutscht, mit wenig Äthanol, Äther und tiefsiedendem Petroläther gewaschen und im Vakuum bei 40 °C getrocknet. Man erhält somit eine Fraktion II des gewünschten Produktes. Zwecks Reinigung werden die vereinigten Fraktionen I und II zunächst in 100 ml Methanol gelöst. Diese Lösung wird mit 900 ml Äthanol verdünnt. Wenig ungelöstes Material wird abfiltriert und das gelbe Filtrat wird im Vakuum bei 40 °C stark eingeengt. Die ausgefallene Substanz wird abgenutscht, mit wenig Äthanol, Äther und tiefsiedendem Petroläther gewaschen. Man erhält beige Titelsubstanz mit $[\alpha]_D^{25} = -244°$ (c = 0,9 in Methanol). Zwecks Umkristallisation und Darstellung hochgereinigten Materials werden 10 g der so erhaltenen Substanz in 50 ml Aceton gelöst. Aus dieser Lösung kristallisiert die Substanz unverzüglich wieder aus. Sie wird abgenutscht, mit Aceton und tiefsiedendem Petroläther gewaschen und über Nacht im Hochvakuum bei 40–45 °C getrocknet. Man erhält beige Reinsubstanz mit $[\alpha]_D^{25} = -249{,}2°$ (c = 1 in Methanol). Das Kernresonanzspektrum und die Mikroanalyse entsprechen der angegebenen Struktur.

Beispiel 3

Herstellung des Natriumsalzes der
(6R,7R)-7-[2-(2-Amino-4-selenazolyl)-2-[(Z)-methoxyimino]-acetamido]-3-methyl-3-oxo-5-thia-1-azabicyclo[4.2.0]oct-2-en-carbonsäure:

8,4 g (6R,7R)-7-[4-Brom-2-[(Z)-methoxyimino]-3-oxo-butyramido]-3-methyl-8-oxo-5-thia-1-azabicyclo[4.2.0]oct-2-en-2-carbonsäure
werden in 160 ml Äthanol suspendiert und mit 2,7 g Selenharnstoff versetzt. Das Gemisch wird 1 Stunde bei 25 °C unter Stickstoff-Begasung und Lichtausschluss gerührt. Die entstandene Lösung wird durch einen Faltenfilter filtriert, um wenig ungelöstes Selen abzutrennen. Das gelbe Filtrat wird mit 22 ml einer 2N Lösung von 2-Äthylcapronsäure-Natriumsalz in Äthylacetat versetzt, wobei das Cephalosporin-Natriumsalz rosagefärbt ausfällt. Nach Zugabe von 160 ml Methanol entsteht wieder eine orange Lösung, welche durch einen Faltenfilter von wenig fein verteiltem, rotem Selen abfiltriert wird. Das gelbe Filtrat wird mit 160 ml Äthanol versetzt und im Vakuum bei 40 °C auf ein Volumen von ca. 100 ml eingeengt. Das dabei auskristallisierte Material wird abgenutscht, mit Äthanol und tiefsiedendem Petroläther gewaschen. Man erhält eine Fraktion I der Titelsubstanz. Die Mutterlauge und der Wasch-Äthanol werden weiter eingedampft, wobei nochmals Substanz auskristallisiert. Diese wird abgenutscht und mit Äthanol und tiefsiedendem Petroläther gewaschen. Man erhält eine Fraktion II der Titelsubstanz. Zwecks Reinigung werden die vereinigten Fraktionen I und II zunächst in 100 ml Methanol gelöst. Diese Lösung wird mit 400 ml Äthanol verdünnt, von wenig fein verteiltem rotem Selen mittels Faltenfilter abfiltriert und im Vakuum bei 40 °C stark eingeengt. Das dabei auskristallisierte Material wird abgenutscht, mit Äthanol und tiefsiedendem Petroläther gewaschen und über Nacht im Hochvakuum bei 40–45 °C getrocknet. Man erhält beige Reinsubstanz mit $[\alpha]_D^{25} = +87°$ (c = 1 in Wasser). Das Kernresonanzspektrum und die Mikroanalyse entsprechen der angegebenen Struktur.

Die oben als Ausgangsmaterial verwendete
(6R,7R)-7-[4-Brom-2-[(Z)-methoxyimino]-3-oxo-butyramido]-3-methyl-8-oxo-5-thia-1-azabicyclo[4.2.0]oct-2-en-2-carbonsäure
kann wie folgt hergestellt werden:

a) Silylierung von 7-Aminodesacetoxycephalosporansäure:

6,7 g 7-Aminodesacetoxycephalosporansäure werden in 400 ml Äthylacetat suspendiert und mit 50 ml N,O-Bis-(trimethylsilyl)-acetamid versetzt. Das Gemisch wird ca. 45 Minuten bei 35–40 °C unter Feuchtigkeitsausschluss gerührt, wobei eine hellgelbe Lösung entsteht. Diese Lösung wird auf −10 bis −15 °C gekühlt und bis zum Einsatz in die Acylierungsreaktion unter Feuchtigkeitsausschluss aufbewahrt.

b) Herstellung von
(Z)-4-Brom-2-methoxyimino-3-oxo-buttersäure-chlorid:
11,2 g (Z)-4-Brom-2-methoxyimino-3-oxo-buttersäure
werden in 150 ml Methylenchlorid bei 0–5 °C mit 10,4 g Phosphorpentachlorid versetzt. Diese Lösung wird 15 Minuten bei 0–5 °C und anschliessend 45 Minuten ohne Kühlung gerührt.

c) Acylierung und Aufarbeitung:
Die nach b) hergestellte Säurechloridlösung wird während ca. 30 Minuten bei −10 °C zur unter a) beschriebenen silylierten 7-Aminodesacetoxycephalosporansäure zugetropft. Das Reaktionsgemisch wird 30 Minuten bei −10 bis 0 °C und ca. 1½ Stunden bei 25 °C gerührt. Danach wird es auf ein Gemisch von 500 ml Wasser und 1000 ml Äthylacetat gegossen. Die organische Phase wird viermal mit je 500 ml Wasser gewaschen, mit Natriumsulfat getrocknet und im Vakuum bei 40 °C stark eingeengt, wobei das Reaktionsprodukt kristallisiert. Dieses wird abgenutscht, mit Äthylacetat, Äther und tiefsiedendem Petroläther gewaschen und über Nacht im Vakuum bei 40 °C getrocknet. Man erhält reine weisse, statisch aufgeladene, kristalline Reinsubstanz mit $[\alpha]_D^{25}$ = +86,8° (c = 1 in Dimethylformamid). Das Kernresonanzspektrum entspricht der angegebenen Struktur.

Beispiel 4
Herstellung von Methylen
(6R,7R)-7-[2-(2-Amino-4-selenazolyl)-2-[(Z)-methoxyimino]acetamido]-3-methyl-8-oxo-5-thia-1-azabicyclo[4.2.0]oct-2-en-2-carboxylat-pivalat:
2,33 g Des Natriumsalzes der
(6R,7R)-7-[2-(2-Amino-4-selenazolyl)-2-[(Z)-methoxyimino]-acetamido]-3-methyl-3-oxo-5-thia-1-azabicyclo[4.2.0]oct-2-en-2-carbonsäure
werden in 25 ml Dimethylformamid gelöst. Diese Lösung wird auf 0–5 °C gekühlt und unter Stickstoff-Begasung mit 2,24 g Pivaloyloxymethyljodid versetzt. Das Reaktionsgemisch wird 30 Minuten bei 0–5 °C gerührt und dann auf 500 ml Wasser gegossen. Die wässrige Phase wird zweimal mit je 250 ml Äthylacetat extrahiert. Die vereinten organischen Phasen werden zweimal mit 100 ml Wasser, zweimal mit 100 ml 5%iger Natriumhydrogencarbonat-Lösung, noch einmal mit 100 ml Wasser gewaschen, über Natriumsulfat getrocknet und im Vakuum bei 40 °C stark eingeengt. Bei der Zugabe von tiefsiedendem Petroläther fällt das Reaktionsprodukt amorph aus. Dieses wird abgenutscht und mit tiefsiedendem Petroläther gewaschen. Man erhält ein gelbliches, amorphes Rohprodukt. Zwecks Reinigung wird dieses an einer Kieselgelsäule mit Äthylacetat als Elutionsmittel chromatographiert. Die die Titelverbindung enthaltenden Fraktionen werden vereinigt und im Vakuum bei 40 °C eingedampft. Nach dem Umfällen aus Äthylacetat/tiefsiedendem Petroläther erhält man schliesslich hellbeige amporphe Reinsubstanz mit $[\alpha]_D^{25}$ = +64,8° (c = 1,07 in Äthylacetat). Das Kernresonanzspektrum und die Mikroanalyse entsprechen der angegebenen Struktur.

Beispiel 5
Herstellung des Natriumsalzes der
(6R,7R)-7-[2-(2-Amino-4-selenazolyl-2-[(Z)-methoxyimino]-acetamido]-3-[[(5-methyl-1,3,4-thiadiazol-2-yl)thio]methyl]-8-oxo-5-thia-1-azabicyclo[4.2.0]oct-2-en-2-carbonsäure:
5,5 g (6R,7R)-7-[4-Brom-2-[(Z)-methoxyimino]-3-oxo-butyramido]-3-[[(5-methyl-1,3,4-thiadiazol-2-yl)thio]methyl]-8-oxo-5-thia-1-azabicyclo[4.2.0]oct-2-en-2-carbonsäure
werden in 80 ml Äthanol gelöst und mit 1,35 g Selenharnstoff versetzt. Das Gemisch wird 1½ Stunden bei 25 °C unter Stickstoff-Begasung und Lichtausschluss gerührt. Es fällt harziges Material aus. Nach Zugabe von 300 ml Methanol werden zur entstandenen Lösung 11 ml einer 2N Lösung von 2-Äthylcapronsäure-Natriumsalz in Äthylacetat gegeben. Es wird von wenig braunem schlammartigem Material abfiltriert. Das gelblich-braune Filtrat wird 30 Minuten bei 25 °C mit Entfärbungskohle gerührt und filtriert. Das hellgelbe Filtrat wird mit 500 ml Äthanol verdünnt und im Vakuum bei 40 °C stark eingeengt, wobei die Substanz auskristallisiert. Letztere wird abgenutscht, mit Äthanol und tiefsiedendem Petroläther gewaschen und über Nacht im Hochvakuum bei 40 °C getrocknet. Man erhält beige Reinsubstanz mit $[\alpha]_D^{25}$ = −53,4° (c = 1 in Wasser). Das Kernresonanzspektrum und die Mikroanalyse entsprechen der angegebenen Struktur.

Die im obigen Verfahren als Ausgangsmaterial verwendete
(6R,7R)-7-[4-Brom-2-[(Z-methoxyimino]-3-oxo-butyramido]-3-[[(5-methyl-1,3,4-thiadiazol-2-yl)thio]methyl]-8-oxo-5-thia-1-azabicyclo-[4.2.0]oct-2-en-2-carbonsäure
wird analog Beispiel 1 hergestellt und als beige amorphe Säure mit ihrer Struktur entsprechendem Kernresonanzspektrum erhalten.

Beispiel 6
Herstellung des Natriumsalzes der
(6R,7R)-7-[2-(2-Amino-4-selenazolyl-2-[(Z)-methoxyimino]-acetamido]-3-[[(1-methyl-1H-tetrazol-5-yl)thio]methyl]-8-oxo-5-thia-1-azabicyclo[4.2.0]oct-2-en-2-carbonsäure:
10,7 g (6R,7R)-7-[4-Brom-2-[(Z)-methoxyimino]-3-oxo-butyramido]-3-[[(1-methyl-1H-tetrazol-5-yl)thio]methyl]-8-oxo-5-thia-1-azabicyclo[4.2.0]oct-2-en-2-carbonsäure
werden in 160 ml Äthanol gelöst und bei 25 °C mit 2,7 g Selenharnstoff versetzt. Das Gemisch wird 1 Stunde bei 25 °C unter Stickstoffbegasung und Lichtausschluss gerührt, wobei harziges Material ausfällt. Nach Zugabe von 200 ml Methanol entsteht eine Lösung, zu welcher 22 ml einer 2N Lösung von 2-Äthylcapronsäure-Natriumsalz in Äthylacetat zugegeben werden. Dabei fällt die gewünschte Verbindung teilweise aus. Es werden weitere 200 ml Methanol zugegeben und von wenig ungelöstem rotem Selen abfiltriert. Das Filtrat wird mit 200 ml Äthanol bis zur leichten Trübung versetzt und im Vakuum bei 40 °C eingeengt. Nachdem 200 ml Destillat übergegangen sind, wird vom ausgefallenen Material abgenutscht. Dieses wird mit Äthanol und tiefsiedendem Petroläther gewaschen. Man erhält eine Fraktion I, welche verworfen wird. Die Mutterlauge wird im Vakuum bei 40 °C weiter eingeengt. Nachdem weitere 250 ml Destillat übergegangen sind, wird vom ausgefallenen Material abgenutscht. Dieses wird mit Äthanol und tiefsiedendem Petroläther gewaschen und über Nacht im Hochvakuum bei 45 °C getrocknet. Man erhält beige Fraktion II mit $[\alpha]_D^{20} = -22,1°$ (c = 1 in Wasser), welche die Titelsubstanz darstellt. Das Kernresonanzspektrum und die Mikroanalyse entsprechen der angegebenen Struktur.

Die im obigen Verfahren als Ausgangsmaterial verwendete

(6R,7R)-7-[4-Brom-2-[(Z)-methoxyimino]-3-oxo-butyramido]-3-[[(1-methyl-1H-tetrazol-5-yl)-thio]methyl]-8-oxo-5-thia-1-azabicyclo-[4.2.0]oct-2-en-2-carbonsäure

wird analog Beispiel 1 hergestellt und als beige amorphe Säure mit $[\alpha]_D^{25} = -27,3°$ (c = 1 in Methanol) mit ihrer Struktur entsprechendem Kernresonanzspektrum erhalten.

Beispiel 7

Herstellung des Natriumsalzes der (6R,7R)-7-[2-(2-Amino-4-selenazolyl)-2-[(Z)-methoxyimino]-acetamido]-8-oxo-3-[[(1,4,5,6-tetrahydro-4-methyl-5,6-dioxo-as-triazin-3-yl)thio]methyl]-5-thia-1-azabicyclo-[4.2.0]oct-2-en-2-carbonsäure:

8,66 g (6R,7R)-7-[4-Brom-2-[(Z)-methoxy-imino]-3-oxo-butyramido]-8-oxo-3-[[(1,4,5,6-tetrahydro-4-methyl-5,6-dioxo-as-triazin-3-yl)thio]methyl]-5-thia-1-azabicyclo-[4.2.0]oct-2-en-2-carbonsäure

werden in 120 ml Äthanol suspendiert und mit 2,03 g Selenharnstoff versetzt. Nach ca. 10 Minuten Rühren ist immer noch eine dicke Suspension vorhanden. Daher werden 120 ml Methanol zugegeben, wobei teilweise eine Lösung entsteht, aus welcher aber wieder Material ausfällt. Das Gemisch wird im Vakuum bei 40 °C eingedampft. Der rötliche Rückstand wird in 300 ml Methanol gelöst und von wenig ungelöstem Selen abfiltriert. Das Filtrat wird mit 20 ml einer 2N Lösung von 2-Äthylcapronsäure-Natriumsalz in Äthylacetat versetzt, wobei das Cephalosporin-Natriumsalz ausfällt. Nun wird das Gemisch mit Methanol auf 1 l verdünnt und die entstandene Lösung von wenig Ungelöstem abfiltriert. Das gelbe Filtrat wird mit 250 ml Äthanol versetzt und im Vakuum bei 40 °C stark eingeengt. Das dabei ausgefallene Material wird abgenutscht, mit Äthanol und tiefsiedendem Petroläther gewaschen und im Vakuum bei 40 °C getrocknet. Man erhält beiges Rohprodukt, welches, zwecks Reinigung in 500 ml Methanol gelöst und mit 250 ml Äthanol versetzt wird. Die trübe Lösung wird durch einen Faltenfilter filtriert und das hellgelbe Filtrat im Vakuum bei 40 °C stark eingeengt. Das ausgefallene Material wird abgenutscht und über Nacht bei 40 °C im Hochvakuum getrocknet. Man erhält beige Reinsubstanz mit $[\alpha]_D^{25} = -37,6°$ (c = 1 in Wasser). Das Kernresonanzspektrum und die Mikroanalyse entsprechen der angegebenen Struktur.

Die im obigen Verfahren als Ausgangsmaterial verwendete

(6R,7R)-7-[4-Brom-2-[(Z)-methoxyimino]-3-oxo-butyramido]-8-oxo-3-[[(1,4,5,6-tetrahydro-4-methyl-5,6-dioxo-as-triazin-3-yl)thio]-methyl]-5-thia-1-azabicyclo[4.2.0]oct-2-en-2-carbonsäure

wird analog Beispiel 1 hergestellt und als beige kristalline Säure mit $[\alpha]_D^{25} = 141,7°$ (c = 0,9% in Dimethylformamid) mit ihrer Struktur entsprechendem Kernresonanzspektrum erhalten.

Beispiel 8

Herstellung des Natriumsalzes der (6R,7R)-3-(Acetoxymethyl)-7-[2-(2-amino-4-selenazolyl)-2-[(Z)-methoxyimino]-acetamido]-8-oxo-5-thia-1-azabicyclo[4.2.0]oct-2-en-2-carbonsäure:

4,8 g (6R,7R)-3-(Acetoxymethyl)-7-[4-brom-2-[(Z)-methoxyimino]-3-oxobutyramido]-8-oxo-5-thia-1-azabicyclo[4.2.0]oct-2-en-2-carbonsäure

werden in 25 ml Methanol gelöst und mit 1,35 g Selenharnstoff versetzt. Das Gemisch wird 1 Stunde bei 25 °C unter Stickstoff-Begasung und Lichtausschluss gerührt. Dann werden 10 ml einer 2N Lösung von 2-Äthylcapronsäure-Natriumsalz in Äthylacetat zugegeben. Wenig ungelöstes rotes Selen wird mittels Faltenfilter abfiltriert. Das gelbe Filtrat wird mit ca. 150 ml Äthanol bis zur leichten Trübung verdünnt. Nach dem Filtrieren mittels Faltenfilter wird das gelbe Filtrat im Vakuum bei 40 °C stark eingeengt, wobei das Cephalosporin-Natriumsalz kristallisiert. Dieses wird abgenutscht, mit Äthanol und tiefsiedendem Petroläther gewaschen und über Nacht im Hochvakuum bei 40 °C getrocknet. Man erhält beige Reinsubstanz mit $[\alpha]_D^{25} = +53,4°$ (c = 1 in Wasser). Das Kernresonanzspektrum und die Mikroanalyse entsprechen der angegebenen Struktur.

Die im obigen Verfahren als Ausgangsmaterial verwendete

(6R,7R)-3-(Acetoxymethyl)-7-[4-brom-2-[(Z)-methoxyimino]-3-oxobutyramido]-8-oxo-5-thia-1-azabicyclo[4.2.0]oct-2-en-2-carbonsäure

wird analog Beispiel 1 hergestellt und als beige kristalline Säure mit $[\alpha]_D^{25} = +59,1°$ (c = 1 in Methanol) mit ihrer Struktur entsprechenden Kernresonanzspektrum und Mikroanalyse erhalten.

Beispiel 9

Herstellung des Dinatriumsalzes der (6R,7R)-3-(Acetoxymethyl)-7-[(Z)-2-(2-amino-4-selenazolyl)-2-[(1-carboxy-1-methyläthoxy)-imino]acetamido]-8-oxo-5-thia-1-azabicyclo-[4.2.0]oct-2-en-2-carbonsäure:

39 g (6R,7R)-3-(Acetoxymethyl)-7-[2-[(Z)-[1-(tert.-butoxycarbonyl)-1-methyläthoxy]-imino]-2-[2-(tritylamino)-4-selenazolyl]-acetamido]-8-oxo-5-thia-1-azabicyclo[4.2.0]-oct-2-en-2-carbonsäure-tert.-butylester

werden in 400 ml Trifluoressigsäure 30 Minuten bei 25 °C gerührt. Die Lösung wird im Vakuum bei 40 °C eingedampft. Das verbleibende Harz wird mit tiefsiedendem Petroläther behandelt. Der resultierende Festkörper wird abgenutscht, mit tiefsiedendem Petroläther gewaschen und über Nacht im Vakuum bei 40 °C getrocknet. Man erhält ein amorphes, gelbliches Gemisch, welches noch unvollständig gespaltenes Material enthält. Zwecks vollständiger Abspaltung der Schutzgruppen wird das isolierte Material in einem Gemisch von 160 ml Trifluoressigsäure und 160 ml 100%ige Ameisensäure gelöst und 1½ Stunden bei 25 °C gerührt. Die Lösung wird im Vakuum bei 40 °C eingedampft. Der Rückstand wird mit 500 ml Äther verrührt, abgenutscht, mit Äther und tiefsiedendem Petroläther gewaschen und im Vakuum bei 40 °C getrocknet. Man erhält gelbliches Trifluoracetat der Titelsubstanz. Zwecks Überführung in das Dinatriumsalz wird letzteres zunächst in 500 ml Methanol gelöst. Diese Lösung wird mit 70 ml einer 2N Lösung von 2-Äthylcapronsäure-Natriumsalz in Äthylacetat und mit 500 ml Äthanol versetzt. Wenig ausgefallenes Material wird abfiltriert und verworfen. Das orange-farbene Filtrat wird im Vakuum bei 40 °C stark eingeengt. Die dabei auskristallisierte Substanz wird abgenutscht, mit Äthanol und tiefsiedendem Petroläther gewaschen und zwei Tage im Hochvakuum bei 40 °C getrocknet. Man erhält gelbliche Titelsubstanz mit $[\alpha]_D^{25} = +36{,}5°$ (c = 1 in Wasser). Das Kernresonanzspektrum und die Mikroanalyse entsprechen der angegebenen Struktur.

Der als Ausgangsmaterial verwendete

(6R,7R)-3-(Acetoxymethyl)-7-[2-[(Z)-[1-(tert.-butoxycarbonyl)-1-methyläthoxy]-imino]-2-[2-(tritylamino)-4-selenazolyl)-acetamido]-8-oxo-5-thia-1-azabicyclo-[4.2.0]oct-2-en-2-carbonsäure-tert.-butylester

kann wie folgt hergestellt werden:

a) Herstellung von
2-Amino-4-selenazolessigsäure-äthylester-(Z)-oxim:

46,13 g Selenharnstoff werden in einem Gemisch von 726 ml Äthanol und 363 ml Wasser gelöst. Unter Stickstoff-Begasung und leichter Kühlung bei 20–25 °C wird diese Lösung portionenweise während ca. 15 Minuten mit 72,6 g

4-Chlor-2-(Z)-hydroxyimino-2-oxo-buttersäure-äthylester

versetzt. Die Reaktionslösung wird 1½ Stunden bei 20–25 °C unter Stickstoffbegasung gerührt, danach wird von wenig ausgeschiedenem, rotem Selen abfiltriert. Das hellgelbe Filtrat wird bei 25 °C mit einer Lösung von 68 g Natriumacetat-trihydrat in 726 ml Wasser versetzt, wobei das pH von 2,8 auf 5,3 steigt und das Reaktionsprodukt auskristallisiert. Das Gemisch wird noch 1½ Stunden bei 25 °C gerührt. Das Produkt wird abgenutscht, mit 250 ml Äthanol und tiefsiedendem Petroläther gewaschen und über Nacht im Vakuum bei 45 °C getrocknet. Man erhält reine Titelsubstanz vom Schmelzpunkt 193–195 °C (Zers.). Das Kernresonanzspektrum und die Mikroanalyse entsprechen der angegebenen Struktur.

b) Herstellung von
2-Tritylamino-4-selenazolbrenztraubensäure-äthylester-(Z)-oxim-hydrochlorid:

72,1 g 2-Amino-4-selenazolessigsäure-äthylester-(Z)-oxim

werden in 150 ml Dimethylformamid gelöst. Zwecks Abtrennung von Spuren Selen wird die Lösung filtriert. Das gelbe Filtrat wird mit 38,25 ml Triäthylamin versetzt, auf −30 °C gekühlt und unter Rühren während ca. 1½ Stunden mit 76,25 g Tritylchlorid bei −30 °C versetzt. Während ca. 1 Stunde lässt man die Temperatur des Reaktionsgemisches auf 20–25 °C steigen, dann wird noch 3 Stunden bei 25 °C gerührt. Das Gemisch wird auf 1 l Äthylacetat gegossen und zweimal mit je 1 l Wasser gewaschen. Die erhaltene bräunliche organische Phase wird mit 1 l 1N wässriger Salzsäure versetzt und gut geschüttelt, wobei das Hydrochlorid ausfällt. Dieses wird abgenutscht und nacheinander mit einer Mischung von 400 ml Wasser und 600 ml Äthylacetat, 2 l Äther und 1 l tiefsiedendem Petroläther gewaschen und über Nacht im Vakuum bei 45 °C getrocknet. Man erhält weisse, kristalline, reine Titelsubstanz vom Schmelzpunkt 193–195 °C (Zers.). Das Kernresonanzspektrum und die Mikroanalyse entsprechen der angegebenen Struktur.

c) Herstellung von
2-Tritylamino-4-selenazolbrenztraubensäure-äthylester-O-[1-(tert.-butoxycarbonyl)-1-methyläthyl]oxim:

108,2 g 2-Tritylamino-4-selenazolbrenztraubensäureäthylester-(Z)-oxim-hydrochlorid

werden in 400 ml Dimethylsulfoxid gelöst, mit 400 ml Aceton sowie 138,3 g granuliertem Kaliumcarbonat und 49 g α-Bromisobuttersäure-tert.-butylester versetzt. Nach 2-stündigem Rühren bei 25 °C werden dem Gemisch noch 70 g pulverisiertes Kaliumcarbonat hinzugefügt. Das Gemisch wird weitere 22 Stunden bei 25 °C und Stickstoff-Begasung gerührt. Die entstandene, dunkle Suspension wird am Vakuum bei 45 °C vom Aceton befreit und auf ein Gemisch von 2 l Wasser und 2 l Äthylacetat gegossen. Die wässrige Phase wird noch mit 1 l Äthylacetat extrahiert und dann verworfen. Die vereinigten, dunklen organischen Phasen werden zweimal mit je 1 l Wasser gewaschen, über Natriumsulfat getrocknet und im Vakuum bei 40 °C eingedampft. Der verbleibende Eindampfrückstand, ein braun-schwarzes Harz, wird aus hochsiedendem Petroläther umkristallisiert. Man erhält die Titelsubstanz als beige-farbene Kristalle vom Schmelzpunkt > 123 °C. Aus dem Kernresonanzspektrum geht hervor, dass die Verbindung als syn/anti-Gemisch (ca. 70 : 30) vorliegt. Die Mikroanalyse entspricht der angegebenen Struktur.

d) Herstellung von
2-Tritylamino-4-selenazolbrenztraubensäure-(Z)-O-[1-(tert.-butoxycarbonyl)-1-methyläthyl]oxim:
90,5 g 2-Tritylamino-4-selenazolbrenztraubensäureäthylester-O-[1-(tert.-butoxycarbonyl)-1-methyläthyl]oxim
werden in 850 ml Methanol mit 140 ml 2N Natronlauge 1½ Stunden unter Rückflussbedingungen erhitzt. Die dunkle Lösung wird im Vakuum bei 40 °C eingedampft. Der feste Einaampfrückstand wird zwischen 1 l Wasser und einem Gemisch von 1 l Äthylacetat und 500 ml Methylenchlorid verteilt und mit 140 ml 3N wässriger Salzsäure sauer gestellt. Die grüne organische Phase wird abgetrennt, von wenig abgeschiedenem Selen abfiltriert, mit Wasser gewaschen, über Natriumsulfat getrocknet und im Vakuum bei 40 °C stark eingeengt. Das auskristallisierte Material wird abgenutscht, mit Äthylacetat und tiefsiedendem Petroläther gewaschen und über Nacht im Vakuum bei 45 °C getrocknet. Man erhält reine Titelsubstanz (reine Z-Form) vom Schmelzpunkt 167–168 °C (Zers.). Das Kernresonanzspektrum und die Mikroanalyse entsprechen der angegebenen Struktur.

e) Herstellung von
(6R,7R)-3-(Acetoxymethyl)-7-[2-[(Z)-[1-(tert.-butoxycarbonyl)-1-methyläthoxy]imino]-2-[2-(tritylamino)-4-selenazolyl]acetamido]-8-oxo-5-thia-1-azabicyclo[4.2.0]oct-2-en-2-carbonsäure-tert.-butylester:
37,1 g 2-Tritylamino-4-selenazolbrenztraubensäure-(Z)-O-[1-(tert.-butoxycarbonyl)-1-methyläthyl]oxim
werden zusammen mit 19,7 g 7-Aminocephalosporansäure-tert.-butylester zu einer Lösung von 5 ml Pyridin in 600 ml Methylenchlorid gegeben. Zu dieser Lösung werden 13,62 g Dicyclohexylcarbodiimid hinzugefügt, und das Gemisch wird 2 Stunden bei 25 °C gerührt. Der auskristallisierte Dicyclohexylharnstoff wird abfiltriert und das gelbe Filtrat im Vakuum bei 40 °C eingedampft. Der Eindampfrückstand wird in 1 l Äthylacetat gelöst. Diese Lösung wird nacheinander mit 500 ml 1N Salzsäure, 500 ml verdünnter wässriger Natriumchlorid-Lösung und 500 ml 5%iger wässriger Natriumhydrogencarbonat-Lösung gewaschen und im Vakuum bei 40 °C eingedampft. Man erhält als Eindampfrückstand einen orange-braunen Schaum. Zur Kristallisation wird dieser in 200 ml Äther gelöst und von wenig ungelöstem Material abfiltriert. Das Filtrat wird 30 Minuten gerührt, wobei die Substanz teilweise auskristallisiert. Nach Zugabe von 300 ml Isopropyläther wird noch 30 Minuten gerührt, um die Kristallisation zu vervollständigen. Die Substanz wird abgenutscht, mit Isopropyläther und tiefsiedendem Petroläther gewaschen und über Nacht im Vakuum bei 40 °C getrocknet. Man erhält praktisch farblose, reine Titelsubstanz mit $[\alpha]_D^{25} = +5°$ (c = 2,2 in Dimethylsulfoxid). Das Kernresonanzspektrum und die Mikroanalyse entsprechen der angegebenen Struktur.

Beispiel 10
Herstellung des Natriumsalzes von
1-[[(6R,7R)-7-[(Z)-2-(2-Amino-4-selenazolyl)-2-[(1-carboxy-1-methyläthoxy)-imino]acetamido]-2-carboxy-8-oxo-5-thia-1-azabicyclo[4.2.0]oct-2-en-3-yl]methyl]-pyridinium-hydroxid (inneres Salz):
8,44 g Dinatriumsalz der
(6R,7R)-3-(Acetoxymethyl)-7-[(Z)-2-(2-amino-4-selenazolyl)-2-[(1-carboxy-1-methyläthoxy)imino]acetamido]-8-oxo-5-thia-1-azabicyclo[4.2.0]oct-2-en-2-carbonsäure
werden zu einer Lösung von 28,48 g Natriumjodid in einem Gemisch von 8,8 ml Wasser und 8 ml Pyridin bei 80 °C gegeben. Das Reaktionsgemisch wird 1 Stunde bei 80 °C gerührt. Die dunkle Lösung wird auf 400 ml Wasser gegossen und im Vakuum bei 45 °C eingedampft. Der Eindampfrückstand wird in 450 ml Wasser gelöst und mit 3N wässriger Salzsäure auf pH 1 gestellt. Das ausgefallene Zersetzungsprodukt wird abgenutscht, mit 100 ml Wasser gewaschen und verworfen. Das hellgelbe Filtrat wird zweimal mit Äthylacetat gewaschen und der pH-Wert mit 3N wässriger Natronlauge auf 6 eingestellt. Dann wird es im Vakuum bei 40 °C stark eingeengt und schliesslich über Nacht lyophilisiert. Zur Entfernung der anorganischen Salze wird das Lyophilisat in 100 ml Wasser gelöst und auf eine Säule mit 1 kg Amberlite XAD-2 gegeben. Mit 3 l Wasser werden die anorganischen Salze herausgewaschen. Dann wird mit 2 l eines Gemisches Wasser-Äthanol 8 : 2 eluiert. Die die Substanz enthaltenden Fraktionen werden im Vakuum bei 40 °C eingeengt und über Nacht lyophilisiert. Man erhält reine Titelsubstanz als beiges, voluminöses Lyophilisat mit $[\alpha]_D^{25} = +3°$ (c = 0,8 in Wasser). Das Kernresonanzspektrum und die Mikroanalyse entsprechen der angegebenen Struktur.

Beispiel 11
Herstellung des Natriumsalzes der
(6R,7R)-3-(Acetoxymethyl)-7-[-2-(2-amino-4-selenazolyl)-2-[(Z)-hydroxyimino]-acetamido]-8-oxo-5-thia-1-azabicyclo[4.2.0]oct-2-en-2-carbonsäure:
9,28 g (6R,7R)-3-(Acetoxymethyl)-7-[4-brom-2-[(Z)-hydroxyimino]acetoacetamido]-8-oxo-5-thia-1-azabicyclo[4.2.0]oct-2-en-2-carbonsäure
werden in 200 ml Methanol gelöst und mit 2,7 g Selenharnstoff versetzt. Das Gemisch wird 1 Stunde bei 25 °C gerührt, dann werden 22 ml einer 2N Lösung des Natriumsalzes der 2-Äthylcapronsäure in Äthylacetat sowie 100 ml Wasser zugegeben. Die Lösung wird von wenig abgeschiedenem Selen abfiltriert. Das gelbe Filtrat wird mit Äthanol

auf 1000 ml bis zur leichten Trübung verdünnt und 30 Minuten gerührt, wobei das Natriumsalz kristallisiert. Dieses wird abgenutscht, mit Äthanol und tiefsiedendem Petroläther gewaschen und über Nacht im Hochvakuum bei 40 °C getrocknet. Man erhält reine, gelbliche Titelsubstanz mit $[\alpha]_D^{25}$ = +70,8° (c = 1 in Wasser). Das Kernresonanzspektrum entspricht der angegebenen Struktur.

Beispiel 12

Herstellung des Natriumsalzes der (6R,7R)-7-[2-[2-Amino-4-selenazolyl]-2-[(Z)-hydroxyimino]acetamido]-3-methyl-8-oxo-5-thia-1-azabicyclo[4.2.0]oct-2-en-2-carbonsäure:

12,2 g (6R,7R)-7-[4-Brom-2-[(Z)-hydroxyimino]-acetoacetamido]-3-methyl-8-oxo-5-thia-1-azabicyclo[4.2.0]oct-2-en-2-carbonsäure

werden in 250 ml Äthanol zusammen mit 4,06 g Selenharnstoff 2 Stunden bei 25 °C gerührt. Die Lösung wird von wenig abgeschiedenem Selen abfiltriert. Das gelbe Filtrat wird mit 35 ml einer 2N Lösung von 2-Äthylcapronsäure-Natriumsalz in Äthylacetat versetzt und 30 Minuten gerührt. Das ausgefallene Natriumsalz wird abgenutscht, mit Äthanol und tiefsiedendem Petroläther gewaschen und über Nacht im Hochvakuum bei 40 °C getrocknet. Man erhält reine beigefarbene, amorphe Titelsubstanz mit $[\alpha]_D^{25}$ = +106° (c = 1 in Wasser). Das Kernresonanzspektrum entspricht der angegebenen Struktur.

Beispiel 13

Herstellung von Trockenampullen für die intramuskuläre Verabreichung:

Es wird in üblicher Weise ein Lyophilisat von 0,5 g des Dinatriumsalzes der (6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)-thio]methyl]-8-oxo-5-thia-1-azabicyclo-[4.2.0]oct-2-en-2-carbonsäure

hergestellt und in eine Ampulle abgefüllt. Vor der Verabreichung wird das Lyophilisat mit 1,7–2,5 ml einer 1%igen wässrigen Lidocainhydrochlorid-Lösung versetzt.

Beispiel 14

Es wird in üblicher Weise eine Gelatine-Steckkapsel folgender Zusammensetzung hergestellt:

| | |
|---|---|
| Methylen(6R,7R)-7-[2-(2-Amino-4-selenazolyl)-2-[(Z)-methoxyimino]-acetamido]-3-methyl-8-oxo-5-thia-1-azabicyclo[4.2.0]oct-2-en-2-carboxylat-pivalat | 500 mg |
| Luviskol (wasserlösliches Polyvinylpyrrolidon) | 20 mg |
| Mannit | 20 mg |
| Talk | 15 mg |
| Magnesiumstearat | 2 mg |
| | 557 mg |

**Patentansprüche**

1. Acylderivate der allgemeinen Formel

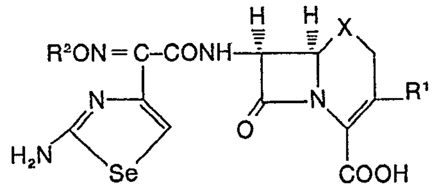

in der $R^1$ Wasserstoff, Methyl, Methoxy, Chlor oder eine Gruppe $-CH_2Y$ darstellt, worin Y einen in der Cephalosporin-Chemie üblichen Rest einer nucleophilen Verbindung bedeutet, worin ferner $R^2$ Wasserstoff oder eine $C_{1-4}$-Alkylgruppe darstellt, die durch die Gruppe $COOR^3$ substituiert sein kann, worin $R^3$ Wasserstoff, ein Kation einer Base oder eine leicht hydrolysierbare Estergruppe bedeutet, und worin X Schwefel, Sauerstoff oder eine der Gruppen –SO– und $-SO_2-$ darstellt, sowie leicht hydrolysierbare Ester, leicht hydrolysierbare Äther und Salze dieser Verbindungen und Hydrate der Verbindungen der Formel I bzw. von deren Estern, Äthern und Salzen.

2. Acylderivate nach Anspruch 1 in der syn-isomeren Form bzw. Gemische, in denen die syn-isomere Form überwiegt.

3. Acylderivate nach Anspruch 1 oder 2, dadurch gekennzeichnet, dass $R^1$ Wasserstoff, Methyl, Methoxy oder Chlor darstellt.

4. Acylderivate nach Anspruch 1, dadurch gekennzeichnet, dass $R^1$ die Gruppe $-CH_2Y$ darstellt, worin Y einen in der Cephalosporin-Chemie üblichen Rest einer nucleophilen Verbindung bedeutet.

5. Acylderivate nach Anspruch 4, dadurch gekennzeichnet, dass Y Acetoxy, Carbamoyloxy oder einen Rest der allgemeinen Formel

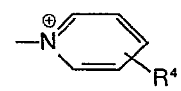

in der $R^4$ Wasserstoff oder Carbamoyl darstellt, bedeutet.

6. Acylderivate nach Anspruch 4, dadurch gekennzeichnet, dass Y einen Rest der allgemeinen Formel

$$-SR^5$$

darstellt, worin $R^5$ einen 5- oder 6-gliedrigen heterocyclischen Ring mit 1–4 Heteroatomen darstellt.

7. Acylderivate nach Anspruch 6, dadurch gekennzeichnet, dass der heterocyclische Ring Sauerstoff, Schwefel, Selen und/oder Stickstoff enthält.

8. Acylderivate nach Anspruch 7, dadurch gekennzeichnet, dass der heterocyclische Ring unsubstituiert oder durch $C_{1-4}$-Alkyl, Halogen, Hydroxy und/oder Oxo substituiert ist.

9. Acylderivate nach Anspruch 8, dadurch gekennzeichnet, dass $R^5$ die 1-Methyl-tetrazol-5-yl-,

2-Methyl-1,3,4-thiadiazol-5-yl-,
1,4,5,6-Tetrahydro-4-methyl-5,6-dioxo-as-triazin-3-yl-,
2,5-Dihydro-6-hydroxy-2-methyl-5-oxo-as-triazin-3-yl- oder
1,2,5,6-Tetrahydro-2-methyl-5,6-dioxo-as-triazin-3-yl-gruppe
darstellt.

10. Acylderivate nach einem der Ansprüche 1–9, dadurch gekennzeichnet, dass $R^2$ Methyl darstellt.

11. Acylderivate nach einem der Ansprüche 1–9, dadurch gekennzeichnet, dass $R^2$ Wasserstoff darstellt.

12. Acylderivate nach einem der Ansprüche 1–9, dadurch gekennzeichnet, dass $R^2$ Carboxymethyl darstellt.

13. Acylderivate nach einem der Ansprüche 1–9, dadurch gekennzeichnet, dass $R^2$ 1-Carboxy-1-methyl-äthyl darstellt.

14. Acylderivate nach einem der Ansprüche 1–9, dadurch gekennzeichnet, dass $R^2$ Pivaloyloxymethoxycarbonylmethyl darstellt.

15. Acylderivate nach einem der Ansprüche 1–14, dadurch gekennzeichnet, dass X Schwefel darstellt.

16. (6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure
sowie Salze dieser Verbindung und Hydrate dieser Verbindung bzw. Salze.

17. (6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)thio]methyl]-8-oxo-5-thia-1-azabicyclo[4.2.0]oct-2-en-2-carbonsäure-5-oxid
sowie Salze dieser Verbindung und Hydrate dieser Verbindung bzw. Salze.

18. (6R,7R)-7-[2-(2-Amino-4-selenazolyl)-2-[(Z)-methoxyimino]-acetamido]-3-methyl-8-oxo-5-thia-1-azabicyclo[4.2.0]oct-2-en-2-carbonsäure
sowie Salze dieser Verbindung und Hydrate dieser Verbindung bzw. Salze.

19. (6R,7R)-7-[2-(2-Amino-4-selenazolyl-2-[(Z)-methoxyimino]-acetamido]-3-[[(5-methyl-1,3,4-thiadiazol-2-yl)thio]-methyl]-8-oxo-5-thia-1-azabicyclo[4.2.0]oct-2-en-2-carbonsäure
sowie Salze dieser Verbindung und Hydrate dieser Verbindung bzw. Salze.

20. (6R,7R)-7-[2-(2-Amino-4-selenazolyl-2-[(Z)-methoxyimino]-acetamido]-3-[[1-methyl-1H-tetrazol-5-yl)-thio]-methyl]-8-oxo-5-thia-1-azabicyclo[4.2.0]oct-2-en-2-carbonsäure
sowie Salze dieser Verbindung und Hydrate dieser Verbindung bzw. Salze.

21. (6R,7R)-7-[2-(2-Amino-4-selenazolyl)-2-[(Z)-methoxyimino)-acetamido]-8-oxo-3-[[(1,4,5,6-tetrahydro-4-methyl-5,6-dioxo-as-triazin-3-yl)thio]methyl]-5-thia-1-azabicyclo[4.2.0]oct-2-en-2-carbonsäure
sowie Salze dieser Verbindung und Hydrate dieser Verbindung bzw. Salze.

22. (6R,7R)-3-Acetoxymethyl)-7-[2-(2-amino-4-selenazolyl)-2-[(Z)-methoxyimino]-acetamido]-8-oxo-5-thia-1-azabicyclo[4.2.0]oct-2-en-2-carbonsäure
sowie Salze dieser Verbindung und Hydrate dieser Verbindung bzw. Salze.

23. (6R,7R)-3-(Acetoxymethyl)-7-[(Z)-2-(2-amino-4-selenazolyl)-2-[(1-carboxy-1-methyläthoxy)imino]acetamido]-8-oxo-5-thia-1-azabicyclo[4.2.0]oct-2-en-2-carbonsäure
sowie Salze dieser Verbindung und Hydrate dieser Verbindung bzw. Salze.

24. 1-[[(6R,7R)-7-[(Z)-2-(2-Amino-4-selenazolyl)-2-[(1-carboxy-1-methyläthoxy)imino]-acetamido]-2-carboxy-8-oxo-5-thia-1-azabicyclo[4.2.0]oct-2-en-3-yl]methyl]pyridinium-hydroxid (inneres Salz)
sowie Salze dieser Verbindung und Hydrate dieser Verbindung bzw. Salze.

25. Leicht hydrolysierbare Ester der Acylderivate der Formel I gemäss einem der Ansprüche 1–24 sowie Salze dieser Ester und Hydrate dieser Ester und Salze.

26. Pivaloyloxymethylester der Acylderivate der Formel I gemäss Anspruch 25 sowie Salze dieser Ester und Hydrate dieser Ester und Salze.

27. Methylen (6R,7R)-7-[2-(2-Amino-4-selenazolyl)-2-[(Z)-methoxyimino]acetamido]-3-methyl-8-oxo-5-thia-1-azabicyclo[4.2.0]oct-2-en-2-carboxylat-pivalat
sowie Salze dieser Verbindung und Hydrate dieser Verbindung bzw. Salze.

28. Acylderivate der allgemeinen Formel

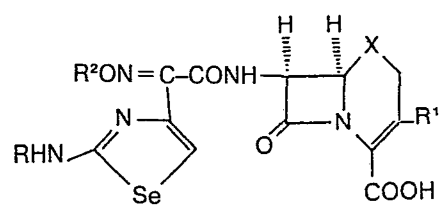

III

in der $R^1$, $R^2$ und X die in Anspruch 1 gegebene Bedeutung haben, R eine abspaltbare Schutzgruppe darstellt und die Carboxygruppe in geschützter Form vorliegen kann.

29. Acylderivate der allgemeinen Formel

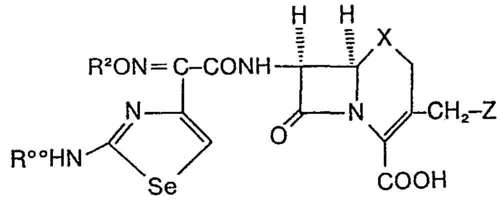

IV + VIII

in der $R^2$ und X die in Anspruch 1 gegebene Bedeutung haben, $R^{oo}$ Wasserstoff oder eine abspaltbare Schutzgruppe und Z eine austretende Gruppe darstellt und die Carboxygruppe durch Salzbildung mit einer anorganischen oder tertiären organischen Base geschützt sein kann.

30. Verbindungen der allgemeinen Formel

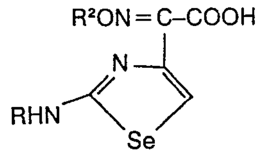

VII

in der R eine abspaltbare Gruppe und $R^2$ Wasserstoff, $C_{1-4}$-Alkyl oder $COOR^3$ $C_{1-4}$-Alkyl, worin $R^3$ Wasserstoff, ein Kation einer Base oder eine leicht hydrolysierbare Estergruppe bedeutet, darstellt,
und reaktionsfähige funktionelle Derivate dieser Verbindungen.

31. Verbindungen der allgemeinen Formel

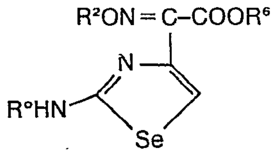

XI+XII

in der $R^0$ Wasserstoff oder eine abspaltbare Gruppe, $R^2$ Wasserstoff, $C_{1-4}$-Alkyl oder $COOR^3$ $C_{1-4}$-Alkyl, worin $R^3$ Wasserstoff, ein Kation einer Base oder eine leicht hydrolysierbare Estergruppe bedeutet und $R^6$ $C_{1-4}$-Alkyl darstellt.

32. Verbindungen gemäss einem der Ansprüche 1–27 als pharmazeutische Wirkstoffe.

33. Verbindungen gemäss einem der Ansprüche 1–27 als pharmazeutische Wirkstoffe zur Behandlung und Prophylaxe von Infektionskrankheiten.

34. Pharmazeutische Präparate, gekennzeichnet durch einen Gehalt an einer Verbindung gemäss einem der Ansprüche 1–27.

35. Pharmazeutische Präparate zur Behandlung und Prophylaxe von Infektionskrankheiten, gekennzeichnet durch einen Gehalt an einer Verbindung gemäss einem der Ansprüche 1–27.

**Claims**

1. Acyl derivatives of the general formula

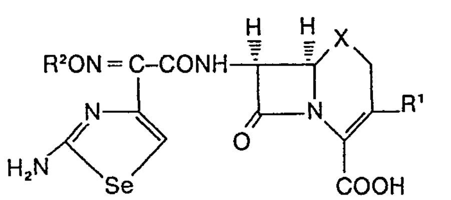

in which $R^1$ represents hydrogen, methyl, methoxy, chlorine or a group $-CH_2Y$, wherein Y signifies a residue of a nucleophilic compound which is usual in cephalosporin chemistry, wherein further $R^2$ represents hydrogen or a $C_{1-4}$-alkyl group which can be substituted by the group $COOR^3$, wherein $R^3$ signifies hydrogen, a cation of a base or a readily hydrolyzable ester group, and wherein X represents sulphur, oxygen or one of the groups $-SO-$ and $-SO_2-$,
as well as readily hydrolyzable esters, readily hydrolyzable ethers and salts of these compounds and hydrates of the compounds of formula I or of their esters, ethers and salts.

2. Acyl derivatives according to claim 1 in the syn-isomeric form or mixtures in which the syn-isomeric form predominates.

3. Acyl derivatives according to claim 1 or 2, characterized in that $R^1$ represents hydrogen, methyl, methoxy or chlorine.

4. Acyl derivatives according to claim 1, characterized in that $R^1$ represents the group $-CH_2Y$, wherein Y signifies a residue of a nucleophilic compound which is usual in cephalosporin chemistry.

5. Acyl derivatives according to claim 4, characterized in that Y signifies acetoxy, carbamoyloxy or a residue of the general formula

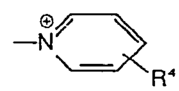

in which $R^4$ represents hydrogen or carbamoyl.

6. Acyl derivatives according to claim 4, characterized in that Y represents a residue of the general formula

$$-SR^5$$

wherein $R^5$ represent a 5- or 6-membered heterocyclic ring with 1–4 hetero atoms.

7. Acyl derivatives according to claim 6, characterized in that the heterocyclic ring contains oxygen, sulphur, selenium and/or nitrogen.

8. Acyl derivatives according to claim 7, characterized in that the heterocyclic ring is unsubstituted or is substituted by $C_{1-4}$-alkyl, halogen, hydroxy and/or oxo.

9. Acyl derivatives according to claim 8, characterized in that $R^5$ represents the
1-methyl-tetrazol-5-yl,
2-methyl-1,3,4-thiadiazol-5-yl,
1,4,5,6-tetrahydro-4-methyl-5,6-dioxo-as-triazin-3-yl,
2,5-dihydro-6-hydroxy-2-methyl-5-oxo-as-triazin-3-yl or
1,2,5,6-tetrahydro-2-methyl-5,6-dioxo-as-triazin-3-yl group.

10. Acyl derivatives according to any one of claims 1–9, characterized in that $R^2$ represents methyl.

11. Acyl derivatives according to any one of claims 1–9, characterized in that $R^2$ represents hydrogen.

12. Acyl derivatives according to any one of claims 1–9, characterized in that $R^2$ represents carboxymethyl.

13. Acyl derivatives according to any one of claims 1–9, characterized in that $R^2$ represents 1-carboxy-1-methyl-ethyl.

14. Acyl derivatives according to any one of claims 1–9, characterized in that $R^2$ represents pivaloyloxymethoxycarbonylmethyl.

15. Acyl derivatives according to any one of claims 1–14, characterized in that X represents sulphur.

16. (6R,7R)-7-[2-(2-Amino-4-selenazolyl)-2-[(Z)methoxyimino]-acetamido]-3-[[(2,5-dihydro-6-hydroxy-2-methyl-5-oxo-as-triazin-3-yl)thio]methyl]-8-oxo-5-thia-1-azabicyclo-[4.2.0]oct-2-ene-2-carboxylic acid
as well as salts of this compound and hydrates of this compound or salts.

17. (6R,7R)-7-[2-(2-Amino-4-selenazolyl)-2-[(Z)-methoxyimino]-acetamido]-3-[[(2,5-dihydro-6-hydroxy-2-methyl-5-oxo-as-triazin-3-yl)thio]methyl]-8-oxo-5-thia-1-azabicyclo-[4.2.0]oct-2-ene-2-carboxylic acid 5-oxide
as well as salts of this compound and hydrates of this compound or salts.

18. (6R,7R)-7-[2-(2-Amino-4-selenazolyl)-2-[(Z)-methoxyimino]-acetamido]-3-methyl-8-oxo-5-thia-1-azabicyclo[4.2.0]oct-2-ene-2-carboxylic acid
as well as salts of this compound and hydrates of this compound or salts.

19. (6R,7R)-7-[2-(2-Amino-4-selenazolyl-2-[(Z)-methoxyimino]-acetamido]-3-[[(5-methyl-1,3,4-thiadiazol-2-yl)thio]-methyl]-8-oxo-5-thia-1-azabicyclo[4.2.0]oct-2-ene-2-carboxylic acid
as well as salts of this compound and hydrates of this compound or salts.

20. (6R,7R)-7-[2-(2-Amino-4-selenazolyl-2-[(Z)-methoxyimino]-acetamido]-3-[[(1-methyl-1H-tetrazol-5-yl)-thio]-methyl]-8-oxo-5-thia-1-azabicyclo[4.2.0]-oct-2-ene-2-carboxylic acid
as well as salts of this compound and hydrates of this compound or salts.

21. (6R,7R)-7-[2-(2-Amino-4-selenazolyl)-2-[(Z)-methoxyimino]-acetamido]-8-oxo-3-[[(1,4,5,6-tetrahydro-4-methyl-5,6-dioxo-as-triazin-3-yl)thio]methyl]-5-thia-1-aza-bicyclo[4.2.0]oct-2-ene-2-carboxylic acid
as well as salts of this compound and hydrates of this compound or salts.

22. (6R,7R)-3-(Acetoxymethyl)-7-[2-(2-amino-4-selenazolyl)-2-[(Z)-methoxyimino]-acet-amido]-8-oxo-5-thia-1-azabicyclo[4.2.0]oct-2-ene-2-carboxylic acid
as well as salts of this compound and hydrates of this compound or salts.

23. (6R,7R)-3-(Acetoxymethyl)-7-[(Z)-2-(2-amino-4-selenazolyl)-2-[(1-carboxy-1-methylethoxy)imino]acetamido-8-oxo-5-thia-1-azabicyclo[4.2.0]oct-2-ene-2-carboxylic acid
as well as salts of this compound and hydrates of this compounds or salts.

24. 1-[[(6R,7R)-7-[(Z)-2-(2-Amino-4-selen-azolyl)-2-[(1-carboxy-1-methylethoxy)imino]-acetamido]-2-carboxy-8-oxo-5-thia-1-aza-bicyclo[4.2.0]oct-2-en-3-yl]methyl]pyridinium hydroxide (internal salt)
as well as salts of this compound and hydrates of this compound or salts.

25. Readily hydrolyzable esters of the acyl derivatives of formula I in accordance with any one of claims 1–24 as well as salts of these esters and hydrates of these esters and salts.

26. Pivaloyloxymethyl esters of the acyl derivatives of formula I in accordance with claim 25 as well as salts of these esters and hydrates of these esters and salts.

27. Methylene (6R,7R)-7-[2-(2-amino-4-selen-azolyl)-2-[(Z)-methoxyimino]acetamido]-3-methyl-8-oxo-5-thia-1-azabicyclo[4.2.0]oct-2-ene-2-carboxylate pivalate
as well as salts of this compound and hydrates of this compound or salts.

28. Acyl derivatives of the general formula

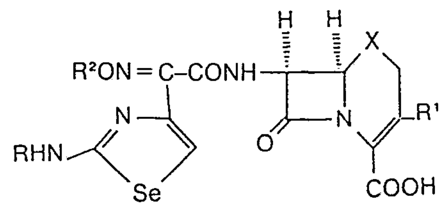

III

in which $R^1$, $R^2$ and X have the significance given in claim 1, R represents a cleavable protecting group and the carboxy group can be present in protected form.

29. Acyl derivatives of the general formula

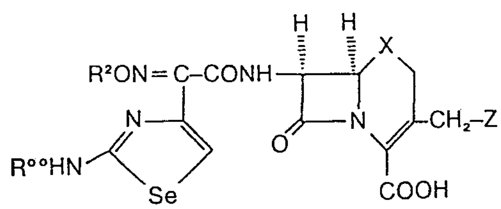

IV + VIII

in which $R^2$ and X have the significance given in claim 1, $R^{oo}$ represents hydrogen or a cleavable protecting group and Z represents a leaving group and the carboxy group can be protected by salt formation with an inorganic or tertiary organic base.

30. Compounds of the general formula

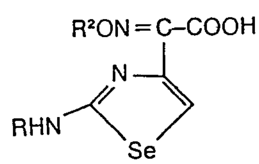

VII

in which R represents a cleavable group and $R^2$ represents hydrogen, $C_{1-4}$-alkyl or $COOR^3$ $-C_{1-4}$-alkyl, wherein $R^3$ signifies hydrogen, a cation of a base or a readily hydrolyzable ester group, and reactive functional derivatives of these compounds.

31. Compounds of the general formula

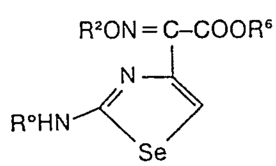

XI + XII

in which $R^0$ represents hydrogen or a cleavable group, $R^2$ represents hydrogen, $C_{1-4}$-alkyl or $COOR^3$-$C_{1-4}$-alkyl, wherein $R^3$ signifies hydrogen,

a cation of a base or a readily hydrolyzable ester group, and $R^6$ represents $C_{1-4}$-alkyl.

32. Compounds in accordance with any one of claims 1–27 as pharmaceutically active substances.

33. Compounds in accordance with any one of claims 1–27 as pharmaceutically active substances for the treatment and prophylaxis of infectious diseases.

34. Pharmaceutical preparations, characterized by a content of a compound in accordance with any one of claims 1–27.

35. Pharmaceutical preparations for the treatment and prophylaxis of infectious diseases, characterized by a content of a compound in accordance with any one of claims 1–27.

**Revendications**

1. Dérivés acylés de formule générale

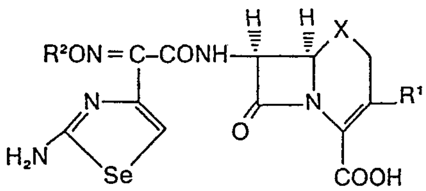

dans laquelle $R^1$ représente l'hydrogène, un groupe méthyle, méthoxy, le chlore ou un groupe $-CH_2Y$ dans lequel Y représente un reste d'un composé nucléophile usuel dans la chimie des céphalosporines, $R^2$ représente l'hydrogène ou un groupe alkyle en $C_1$–$C_4$ qui peut être substitué par le groupe $COOR^3$ dans lequel $R^3$ représente l'hydrogène, un cation d'une base ou un groupe ester facile à hydrolyser, et X représente le soufre, l'oxygène ou l'un des groupes $-SO-$ et $-SO_2-$, ainsi que leurs esters faciles à hydrolyser, leurs éthers faciles à hydrolyser et les sels de ces composés et hydrates des composés de formule I et de leurs esters, éthers et sels.

2. Dérivés acylés selon la revendication 1, sous la forme isomère syn et mélanges dans lesquels la forme isomère syn est prépondérante.

3. Dérivés acylés selon la revendication 1 ou 2, caractérisés en ce que $R^1$ représente l'hydrogène, un groupe méthyle ou méthoxy ou le chlore.

4. Dérivés acylés selon la revendication 1, caractérisés en ce que $R^1$ représente le groupe $-CH_2Y$ dans lequel Y est un reste d'un composé nucléophile usuel dans la chimie des céphalosporines.

5. Dérivés acylés selon la revendication 4, caractérisés en ce que Y représente un groupe acétoxy, carbamoyloxy ou un reste de formule générale

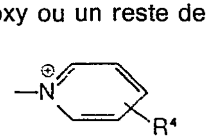

dans laquelle $R^4$ représente l'hydrogène ou un groupe carbamoyle.

6. Dérivés acylés selon la revendication 4, caractérisés en ce que Y représente un reste de formule générale

$$-SR^5,$$

dans laquelle $R^5$ représente un hétérocycle à 5 ou 6 chaînons contenant 1 à 4 hétéroatomes.

7. Dérivés acylés selon la revendication 6, caractérisés en ce que l'hétérocycle contient de l'oxygène, du soufre, du sélénium et/ou de l'azote.

8. Dérivés acylés selon la revendication 7, caractérisés en ce que l'hétérocycle est non substitué ou substitué par des groupes alkyle en $C_1$–$C_4$, des halogènes, des groupes hydroxy et/ou oxo.

9. Dérivés acylés selon la revendication 8, caractérisés en ce que $R^5$ représente le groupe
1-méthyltétrazole-5-yle,
2-méthyl-1,3,4-thiadiazole-5-yle,
1,4,5,6-tétrahydro-4-méthyl-5,6-dioxo-as-triazine-3-yle,
2,5-dihydro-6-hydroxy-2-méthyl-5-oxo-as-triazine-3-yle ou
1,2,5,6-tétrahydro-2-méthyl-5,6-dioxo-as-triazine-3-yle.

10. Dérivés acylés selon l'une des revendications 1 à 9, caractérisés en ce que $R^2$ représente un groupe méthyle.

11. Dérivés acylés selon l'une des revendications 1 à 9, caractérisés en ce que $R^2$ représente l'hydrogène.

12. Dérivés acylés selon l'une des revendications 1 à 9 caractérisés en ce que $R^2$ représente un groupe carboxyméthyle.

13. Dérivés acylés selon l'une des revendications 1 à 9 caractérisés en ce que $R^2$ représente le groupe 1-carboxy-1-méthyl-éthyle.

14. Dérivés acylés selon l'une des revendications 1 à 9 caractérisés en ce que $R^2$ représente le groupe pivaloyloxyméthoxy-carbonylméthyle.

15. Dérivés acylés selon l'une des revendications 1 à 14, caractérisés en ce que X représente le soufre.

16. L'acide (6R,7R)-7-[2-(2-amino-4-sélénazolyl)-2-[(Z)-méthoxy-imino]-acétamido]-3-[[(2,5-dihydro-6-hydroxy-2-méthyl-5-oxo-as-triazine-3-yl)thio]méthyl]-8-oxo-5-thia-1-azabicyclo[4.2.0]octa-2-ène-2-carboxylique
et les sels de ce composé et hydrates de ce composé et de ses sels.

17. Le 5-oxyde de l'acide (6R,7R)-7-[2-(2-amino-4-sélénazolyl)-2-[(Z)-méthoxy-imino]-acétamido]-3-[[(2,5-dihydro-6-hydroxy-2-méthyl-5-oxo-as-triazine-3-yl)thio]-méthyl]-8-oxo-5-thia-1-azabicyclo[4.2.0]-octa-2-ène-2-carboxylique,
les sels de ce composé et hydrates de ce composé et de ses sels.

18. L'acide (6R,7R)-7-[2-(2-amino-4-sélénazolyl)-2-[(Z)-méthoxy-imino]-acétamido]-3-méthyl-8-oxo-5-thia-1-azabicyclo[4.2.0]-octa-2-ène-2-carboxylique
et les sels de ce composé et hydrates de ce composé et de ses sels.

19. L'acide (6R,7R)-7-[2-(2-amino-4-sélénazolyl)-2-[(Z)-méthoxy-imino]acétamido]-3-[[(5-méthyl-1,3,4-thiadiazole-2-yl)thio]-méthyl]-8-oxo-5-thia-1-azabicyclo[4.2.0]-octa-2-ène-2-carboxylique
et les sels de ce composé et hydrates de ce composé et de ses sels.

20. L'acide (6R,7R)-7-[2-(2-amino-4-sélénazolyl)-2-[(Z)-méthoxy-imino]-acétamido]-3-[[(1-méthyl-1H-tétrazole-5-yl)thio]-méthyl]-8-oxo-5-thia-1-azabicyclo[4.2.0]-octa-2-ène-2-carboxylique
et les sels de ce composé et hydrates de ce composé et de ses sels.

21. L'acide (6R,7R)-7-[2-(2-amino-4-sélénazolyl)-2-[(Z)-méthoxy-imino]-acétamido]-8-oxo-3-[[(1,4,5,6-tétrahydro-4-méthyl-5,6-dioxo-as-triazine-3-yl)thio]-méthyl]-5-thia-1-azabicyclo[4.2.0]octa-2-ène-2-carboxylique
et les sels de ce composé et hydrates de ce composé et de ses sels.

22. L'acide (6R,7R)-3-(acétoxyméthyl)-7-[2-(2-amino-4-sélénazolyl)-2-[(Z)-méthoxy-imino]-acétamido]-8-oxo-5-thia-1-aza-bicyclo[4.2.0]octa-2-ène-2-carboxylique
et les sels de ce composé et hydrates de ce composé et de ses sels.

23. L'acide (6R,7R)-3-(acétoxyméthyl)-7-[(Z)-2-(2-amino-4-sélénazolyl)-2-[(1-carboxy-1-méthyléthoxy)imino]acétamido]-8-oxo-5-thia-1-azabicyclo[4.2.0]octa-2-ène-2-carboxylique
et les sels de ce composé et hydrates de ce composé et de ses sels.

24. L'hydroxyde (sel interne) de 1-[[(6R,7R)-7-[(Z)-2-(2-amino-4-sélénazolyl)-2-[(1-carboxy-1-méthyléthoxy)imino]-acétamido]-2-carboxy-8-oxo-5-thia-1-aza-bicyclo[4.2.0]octa-2-ène-3-yl]méthyl]-pyridinium
et les sels de ce composé et hydrates de ce composé et de ses sels.

25. Esters faciles à hydrolyser des dérivés acylés de formule I selon l'une des revendications 1 à 24, et sels de ces esters et hydrates de ces esters et sels.

26. Esters pivaloyloxyméthyliques des dérivés acylés de formule I selon la revendication 25 et sels de ces esters et hydrates de ces esters et sels.

27. Le (6R,7R)-7-[2-(2-amino-4-sélénazolyl)-2-[(Z)-méthoxyimino]acétamido]-3-méthyl-8-oxo-5-thia-1-azabicyclo[4.2.0]-octa-2-ène-2-carboxylate-pivalate
de méthylène et les sels de ce composé et hydrates de ce composé et de ses sels.

28. Dérivés acylés de formule générale:

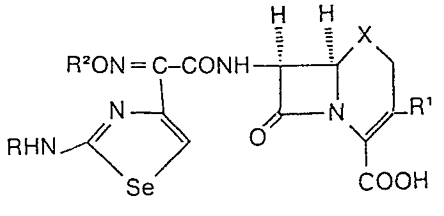

III

dans laquelle $R^1$, $R^2$ et X ont les significations indiquées dans la revendication 1, R représente un groupe protecteur éliminable et le groupe carboxy peut être à l'état protégé.

29. Dérivés acylés de formule générale:

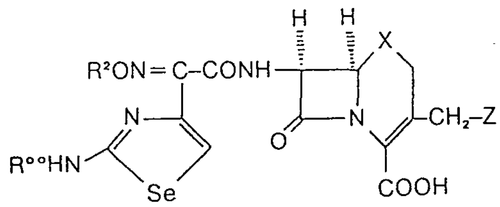

IV + VIII

dans laquelle $R^2$ et X ont les significations indiquées dans la revendication 1, $R^{00}$ représente l'hydrogène ou un groupe protecteur éliminable et Z représente un groupe éliminable, et le groupe carboxy peut être protégé par salification avec une base minérale ou organique tertiaire.

30. Composés de formule générale

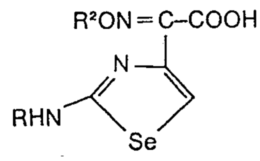

VII

dans laquelle R représente un groupe éliminable et $R^2$ représente l'hydrogène, un groupe alkyle en $C_1$–$C_4$ ou $COOR^3$-alkyle en $C_1$–$C_4$, $R^3$ représentant l'hydrogène, un cation d'une base ou un groupe ester facile à hydrolyser, et dérivés fonctionnels réactifs de ces composés.

31. Composés de formule générale

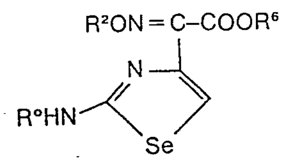

XI + XII

dans laquelle $R^0$ représente l'hydrogène ou un groupe éliminable, $R^2$ représente l'hydrogène, un groupe alkyle en $C_1$–$C_4$ ou $COOR^3$-alkyle en $C_1$–$C_4$, $R^3$ représentant l'hydrogène, un cation d'une base ou un groupe ester facile à hydrolyser et $R^6$ un groupe alkyle en $C_1$–$C_4$.

32. Composés selon l'une des revendications 1 à 27 en tant que substances actives pharmaceutiques.

33. Composés selon l'une des revendications 1 à 27 en tant que substances actives pharmaceutiques pour le traitement et la prophylaxie de maladies infectieuses.

34. Compositions pharmaceutiques caractérisées en ce qu'elles contiennent un composé selon l'une des revendications 1 à 27.

35. Compositions pharmaceutiques pour le traitement et la prophylaxie de maladies infectieuses, caractérisées en ce qu'elles contiennent un composé selon l'une des revendications 1 à 27.